(19) Europäisches Patentamt
European Patent Office
Office européen des brevets

(11) **EP 2 475 296 B1**

(12) **EUROPEAN PATENT SPECIFICATION**

(45) Date of publication and mention
of the grant of the patent:
**17.05.2017 Bulletin 2017/20**

(21) Application number: **10816189.4**

(22) Date of filing: **10.09.2010**

(51) Int Cl.:
*A61B 5/024* (2006.01)   *A61B 5/01* (2006.01)
*A61B 5/00* (2006.01)   *A61N 1/36* (2006.01)
*A61B 5/11* (2006.01)

(86) International application number:
**PCT/US2010/048497**

(87) International publication number:
**WO 2011/032016 (17.03.2011 Gazette 2011/11)**

(54) **IMPROVED DIAGNOSTIC SENSORS FOR GASTROINTESTINAL STIMULATION OR MONITORING DEVICES**

VERBESSERTE DIAGNOSESENSOREN ZUR MAGEN-DARM-STIMULATION ODER FÜR ÜBERWACHUNGSVORRICHTUNGEN

CAPTEURS DE DIAGNOSTIC POUR LA STIMULATION GASTRO-INTESTINALE OU DISPOSITIFS DE SURVEILLANCE AMÉLIORÉS

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **10.09.2009 US 241154 P**

(43) Date of publication of application:
**18.07.2012 Bulletin 2012/29**

(73) Proprietor: **Intrapace, Inc.**
**Mountain View, CA 94043 (US)**

(72) Inventors:
• **PROVINCE, Rose**
**San Jose**
**California 95112 (US)**
• **POTOSKY, John, C.**
**San Jose**
**California 95136 (US)**

• **BRYNELSEN, Charles, R.**
**Menlo Park**
**California 94025 (US)**

(74) Representative: **Clark, Jane Anne et al**
**Mathys & Squire LLP**
**The Shard**
**32 London Bridge Street**
**London SE1 9SG (GB)**

(56) References cited:
**WO-A2-2007/102134**   **US-A1- 2002 156 351**
**US-A1- 2005 065 571**   **US-A1- 2005 113 703**
**US-A1- 2005 171 410**   **US-A1- 2005 172 311**
**US-A1- 2006 020 177**   **US-A1- 2006 064 037**
**US-A1- 2007 173 705**   **US-A1- 2007 255 334**
**US-A1- 2008 161 715**   **US-A1- 2008 183 090**
**US-B1- 6 183 425**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

Field of Invention:

The present invention relates generally to a system for treating an eating disorder as defined in claim 1.

Since the mid-seventies, the prevalence of obesity has increased sharply for both adults and children. These increasing rates raise concern because of their implications for Americans' health. Being overweight or obese may increase the risk of many diseases and health conditions, including: hypertension, dyslipidemia (for example, high total cholesterol or high levels of triglycerides), type 2 diabetes, coronary heart disease, stroke, gallbladder disease, osteoarthritis, sleep apnea and respiratory problems, and some cancers (such as endometrial, breast, and colon).

Obesity and its associated health problems have a significant economic impact on the U.S. health care system. Medical costs associated with excess weight and obesity may involve direct and indirect costs. Direct medical costs may include preventive, diagnostic, and treatment services related to obesity. Indirect costs relate to morbidity and mortality costs. Morbidity costs are defined as the value of income lost from decreased productivity, restricted activity, absenteeism, and bed days. Mortality costs are the value of future income lost by premature death.

Many therapies are currently being investigated for treatment of obesity and diseases associated with obesity. To date, the widely used obesity treatments have not been shown to be ideal, particularly for those afflicted with severe obesity. The approaches that have been proposed range from lifestyle coaching to major surgical therapies. Unfortunately, patient compliance and the accuracy with which patients report their own activities can significantly limit the effectiveness of coaching and support groups. While surgical approaches can limit the capacity of the patient's food intake over a set amount of time regardless of compliance, quite severe surgical modifications may have to be imposed to achieve the desired result. Notwithstanding that, as a group, obese patients may be highly motivated to find a solution to help them lose weight and to improve their health, obese individuals will often exhibit behavior which circumvents or limits the efficacy of therapies so that effective surgical approaches may have to significantly restrict gastrointestinal function, while more moderate approaches may not achieve the desired results. Nonetheless, improved awareness of obesity's role in increasing the incidence of other serious health issues is contributing to overweight consumers' desire to take a more active role in the management of their weight, lifestyle and health.

Because calories not used during activity are stored as body fat, balancing activity levels and ingestion events are important factors in managing weight and health. One of the most accurate methods to assess energy expenditure is through the measurement of oxygen consumption and carbon dioxide production. Unfortunately, this method, called indirect calorimetry, can be inconvenient when trying to measure energy expenditure in free living situations. In order to determine energy expenditure for patients in free-living situations, researches have turned to a variety of alternatives, but none of these research systems have found widespread use in a clinical setting. Consumer products that have been proposed may suffer from compliance and/or accuracy limitations, thereby limiting their efficacy for clinical treatment of morbid obesity and other eating disorders.

Therefore, it would be desirable to provide devices, systems and methods that can effectively monitor activity level, energy expenditure and caloric intake of patients suffering from obesity or eating disorders. Systems that are not fully dependant on patient compliance would be more objective providing a clearer picture of actual patient activities. It would also be desirable to provide improved assessment of a patient's health and actual behavior. Ideally, such a system would provide a patient and/or their health care professional access to the information collected about the patient's exercise and eating habits. This information could be used to monitor the patient's progress and present actual behavior-based information to the patient for effective behavior modification and greater success in achieving weight loss or health goals. Furthermore, since gastric banding and bypass results are affected by patient compliance as well, the actual behavior based information may be a useful tool as an adjunct to these therapies.

Background Art:

Studies relating to monitoring activity levels and estimating energy expenditure are described, for example, in: Committee, P.A.G.A., Physical Activity Guidelines Advisory Committee Report, 2008, U.S.D.o.H.a.H. Services, Editor. 2008: Washington, DC; Bouten, C., A Triaxial Accelerometer and Portable Data Processing Unit for the Assessment of Daily Physical Activity. IEEE Transactions on Biomedical Engineering 1997. 44(3); Chen, K.Y. and M. Sun, Improving energy expenditure estimation by using a triaxial accelerometer. J Appl Physiol, 1997. 83(6): p. 2112-22; Plasqui, G., et al., Measuring free-living energy expenditure and physical activity with triaxial accelerometry. Obes Res, 2005. 13(8): p. 1363-9; Schutz, Y., et al., A new accelerometric method to assess the daily walking practice. Int J Obes Relat Metab Disord, 2002. 26(1): p. 111-8; McCrory, M.A., et al., Between-day and within-day variability in the relation between heart rate and oxygen consumption: effect on the estimation of energy expenditure by heart-rate monitoring. Am J Clin Nutr, 1997. 66(1): p. 18-25; Strath, S.J., et al., Evaluation of heart rate as a method for assessing moderate intensity physical activity. Med Sci Sports Exerc, 2000. 32(9 Suppl): p. S465-70; Ceesay, S.M., et al., The use of heart rate monitoring in the estimation of energy expenditure: a validation study using indirect whole-body calorimetry. Br J Nutr, 1989. 61(2): p. 175-86; Crouter, S.E., J.R. Churilla, and D.R. Bassett, Jr., Accuracy of the for the assessment of energy expenditure in adults. Eur J Clin Nutr, 2008. 62(6): p. 704-11.; Moon, J.K. and N.F. Butte, Combined heart rate and activity improve estimates of oxygen consumption and carbon dioxide production rates. J Appl Physiol, 1996. 81(4): p. 1754-61; Strath, S.J., et al., Simultaneous heart rate-motion sensor technique to estimate energy expenditure. Med Sci Sports Exerc, 2001. 33(12): p. 2118-23; Strath, S.J., et al., Validity of the simultaneous heart rate-motion sensor technique for measuring energy expenditure. Med Sci Sports Exerc, 2002. 34(5): p. 888-94; Treuth, M.S., A.L. Adolph, and N.F. Butte, Energy expenditure in children predicted from heart rate and activity calibrated against respiration calorimetry. Am J Physiol, 1998. 275(1 Pt 1): p. E 12-8; J akicic, J.M., et al., *Evaluation of the Sense Wear Pro Armband to assess energy expenditure during exercise.*

Med Sci Sports Exerc, 2004. 36(5): p. 897-904; St-Onge, M., et al., Evaluation of a portable device to measure daily energy expenditure in free-living adults. Am J Clin Nutr, 2007. 85(3): p. 742-9; Papazoglou, D., et al., Evaluation of a multisensor armband in estimating energy expenditure in obese individuals. Obesity (Silver Spring), 2006. 14(12): p. 2217-23; Bouten, C.V., et al., Assessment of energy expenditure for physical activity using a triaxial accelerometer. Med Sci Sports Exerc, 1994. 26(12): p. 1516-23; Pate, R.R., et al., Physical activity and public health. A recommendation from the Centers for Disease Control and Prevention and the American College of Sports Medicine. JAMA, 1995. 273(5): p. 402-7; Levine, J.A., et al., Jnterindividual variation in posture allocation: possible role in human obesity. Science, 2005. 307(5709): p. 584-6; Mac William, Postural Effects on Heart-Rate and Blood-Pressure. Exp Physiol, 1933. XXIII; Crouter, S.E. and D.R. Bassett, Jr., A new 2-regression model for the Actical accelerometer. Br J Sports Med, 2008. 42(3): p. 217-24; Kim, D., et al., Detection of subjects with higher self-reporting stress scores using heart rate variability patterns during the day. Conf Proc IEEE Eng Med Biol Soc, 2008. 2008: p. 682-5; and Yoshiuchi, K., et al., Yearlong physical activity and depressive symptoms in older Japanese adults: cross-sectional data from the Nakanojo study. Am J Geriatr Psychiatry, 2006. 14(7): p. 621-4. Methods and devices for gastric treatment devices are described in U.S. Patent Numbers 4,178,105, 7,020,531, 7,120,498, 7,509,174, 7,509,175, 7,054,690 and 7,430,450, for example. Commercially available 25 fitness monitors include GRUVE™ by MUVE®, Inc., (Minneapolis, MN), and Forerunner® 305 by Garmin®, Inc. (Olathe, KS).

US 2007/0173705 describes a system for monitoring and reporting the activity level and caloric expenditure of an individual. The system has sensors in electronic communication with a wearable device and a program is programmed to communicate with the sensor and a computing device to obtain activity data from movement related data.

US2007/0255334 describes techniques that allow an implantable device to sense gastric data and activity data from a patient, and estimate the patient's amount of energy consumed and energy expended based on the sensed data. A system provides feedback to the patient, a family member, or a doctor about the patient's energy consumed, energy expended, and net energy.

BRIEF SUMMARY OF THE INVENTION

The present invention is set out in the appended claims. Described herein is monitoring the health and energy expenditure of a living body, treating eating disorders or treating obese patients. Exemplary embodiments use improved diagnostic sensors and/or treatments for gastrointestinal stimulation and monitoring devices. Although some embodiments make specific reference to treatment for obesity, the system and methods described herein may be applicable to other treatments seeking patient behavior modification, and particularly eating disorders and other disorders in which presenting feedback regarding patients' actual eating and/or exercise habits is desired. One specific example would be alcohol consumption for treatment-resistant alcoholics.

Described herein is a method for monitoring the health of a living body. The method includes implanting sensors within the body. The sensors generate signals correlating to energy expenditure of the body. A first set of signals transmitted during a first portion of a time span indicate a first rate of energy expenditure of the body while a second set of signals transmitted during a second portion of the time span indicate a second rate of energy expenditure. The second energy expenditure is greater than the first energy expenditure. The overall caloric energy expenditure of the body for the time span is calculated using the signals so that the calculated overall energy expenditure correlates to the first energy expenditure during the first time portion combined with the second energy expenditure during the second time portion. The calculated overall energy expenditure can be displayed or the body can be treated in response to the calculated overall energy expenditure. The energy expenditure is preferably displayed such that improvement in actual behavior can be monitored.

In some embodiments, the sensors transmit the signals from within the body for more than a month. The calculated overall energy expenditure is displayed and/or the body is treated in response to the overall energy expenditure to induce a desired improvement in the health of the body while the sensors are implanted in the body.

In some embodiments, the sensors transmit the signals from within the body of a patient with an eating disorder for multiple months. The calculated overall energy expenditure is displayed and/or the body is treated in response to the overall energy expenditure to mitigate the patient's eating disorder. Furthermore, the display of calculated overall energy expenditure allows physicians to monitor compliance with an exercise prescription.

Sensors are helpful to perceive and monitor various changes in body parameters. In some embodiments, the patient data is transmitted from the sensor(s) via wireless electrical signals to the processor.

In some embodiments, the body's activity level during the first portion of time is determined to be within a sedentary range and the activity level during the second time portion is determined to be within an active range. Overall energy expenditure is calculated using a sedentary regression model for the first time portion and an active regression model, which is different from the sedentary regression model, for the second time portion.

In other embodiments, the intensities of activity levels are determined in response to a comparison between an integration of accelerometer signals and a threshold. For each time portion, the overall energy expenditure corresponds to an intensity of the activity level multiplied by a time period of the associated time portion. Each intensity corresponds to a combination of an accelerometer-based intensity portion with a heart-rate based intensity portion. The relative contributions of the intensity portions vary according to the determined range of intensity level.

In some embodiments, the activity level during the second time portion includes a vigorous activity level. At least one portion of the time span is associated with a moderate activity level and at least one portion of the time span is associated with a light activity level. These activity levels are identified. For each portion of a time span, associated energy expenditures are calculated using an energy expenditure intensity factor of a time period associated with the time portion based on an activity level of that time period. The overall energy expenditure is calculated by tallying the energy expenditure portions throughout the time span.

In other embodiments, the sensors include an implanted core body temperature sensor. A sleeping period can be identified in response to the core body temperature. The overall energy expenditure corresponds to a sleeping energy expenditure portion which is determined by using the sleeping period, an active energy expenditure portion determined for the second time portion (associated with an active time period), and a sedentary waking energy expenditure portion determined for the first time portion (associated with a sedentary awake time period).

In additional embodiments, the core body temperature sensor is disposed in a stomach cavity of the body so that the core body temperature sensor serves as an ingestion sensor. Ingestion is identified using rapid temperature changes. Sleep is identified using a downward shift in core body temperature while the activity is consistent with sleep.

In other embodiments, ingestion into the body is determined using an ingestion sensor (optionally a temperature sensor) disposed in a stomach cavity of the body. The core body temperature sensor is implanted in another location than the ingestion sensor, to ensure that the core body temperature sensor is thermally separated from the ingestion sensor. One potential implantation location for the core body temperature sensor includes the implantable device housing (CAN) or implantable pulse generator (IPG).

In still other embodiments, a quantified caloric content of ingestion into the body during the time span is determined. The overall energy expenditure and the caloric ingestion to the body while the sensors are implanted within the body is graphically displayed via a graphical interface to influence healthy behavior by the patient.

In other embodiments, various body tissues are stimulated to inhibit unhealthy ingestion into the body in response to the signals.

In additional embodiments, a tilt sensor collects data during various postural allocations of an obese patient and is configured to evaluate non-exercise activity thermogenesis (NEAT) related energy expenditure.

Described herein is a method for monitoring the total energy expenditure over a period of time. The method includes coupling an accelerometer to the body to generate accelerometer signals in response to body movements. A heart rate sensor is coupled to the body to generate heart rate signals in response to the body's heart rate. A core body temperature sensor is also coupled to the body to generate temperature signals in response to a core body temperature. The total energy expenditure over a period of time is calculated using the signals so that, for a plurality of portions of the time span, associated energy expenditure rates are identified from the signals.

In some embodiments, the overall energy expenditure of the body is calculated by identifying multiple activity levels of the body from the signals and integrating the time period intervals or multiplying the time spent at each activity level times an associated energy expenditure rate. The identified activity levels include sleep and the sleep period is determined using a combination of an individual's heart rate, temperature and triaxial accelerometer activity level signals.

Described herein is a system for monitoring the health of a living body. The system includes a plurality of implanted sensors. Each sensor generates signals that correlate to energy expenditure of the body when they are implanted. A processor is attached to the sensors so that the processor, in response to the signals, determines a calculated overall caloric energy expenditure. In response to a first set of signals during a first portion of a time span, the processor determines a first rate of energy expenditure of the body. In response to a second set of the signals during a second portion of the time span, the processor determines a second rate of energy expenditure greater than the first rate of energy expenditure and calculates the overall energy expenditure by combining the energy expenditure rates with their associated time periods. A display or treatment-signal applicator is attached to the processor to receive the overall energy expenditure and display the overall energy expenditure of the body and/or treat the body.

In some embodiments, each sensor transmits signals from within the body for at least a month. While the sensors are implanted in the body and in response to the overall energy expenditure, a display shows the calculated overall energy expenditure performed. This is designed to induce a desired improvement in the health of the body.

In some embodiments, each sensor transmits signals from within the body for multiple months. While the sensors are implanted in the body of a patient with an eating disorder and in response to the calculated overall energy expenditure, a display shows the calculated overall hourly, daily, and weekly energy expended. This is intended to mitigate the eating disorder.

In other embodiments, the implanted sensors include an accelerometer, a heart rate sensor, and a body temperature sensor. In still other embodiments, the accelerometer includes a triaxial (3D) accelerometer.

In some embodiments, the processor uses signals from a triaxial (3D) accelerometer, heart rate and temperature sensors to identify a sleeping, sedentary waking and awake periods. Overall energy expenditure is determined by tallying the sleeping energy expenditure portion determined by the sleeping period, a sedentary waking energy expenditure portion determined by the sedentary waking portion and an active energy expenditure portion determined for the awake time portion.

In some embodiments, the temperature sensor is disposed in the stomach cavity of the body so that the sensor serves as an ingestion sensor. The ingestion sensor detects food consumption based on a rapid shift in homeostatic temperature where the activity is consistent with the eating period. An alternative embodiment of this sensor filters out ingestion events.

In other embodiments, the caloric content of ingestion into the body during the time span is quantified. Overall energy expenditure and caloric ingestion into the body is graphically displayed while the sensors are implanted within the body.

In many embodiments the treatment signal applicator will include a stimulator to stimulate tissues of the body to inhibit unhealthy ingestion (e.g. excessive quantities, foods with excessive sugar or fat contents, or both) into the body in response to the signals. Suitable signal applicators may apply electrical or other stimulation signals.

Described herein is a system for treating an eating disorder of a patient. The system includes an accelerometer coupled to the body to generate accelerometer signals in response to body movement. A heart rate sensor is also coupled to the body to generate heart rate signals in response to the body's heart rate. A core body temperature sensor is coupled to the body. The core body temperature sensor generates temperature signals in response to a core temperature of the body. Finally, a processor is attached to the sensors so that the processor, in response to the signals, calculates an overall caloric energy expenditure of the body for a time span using the signals. In this way, associated energy expenditure rates are identified from the signals for multiple time spans. In other embodiments, the sensor data includes ingestion and activity level information.

In some embodiments, numerous activity levels of the body are identified from the signals. This includes determining sleep onset based on reduced levels of accelerometer based activity, heart rate, and core body temperature. In other embodiments, the accelerometer of the present system is implanted in the body of the patient.

In more embodiments, the transmitted signals of the accelerometer are sampled at adaptive and varying rates to conserve system power requirements and increase resolution of body movement. In additional embodiments, the accelerometer is an omni-directional accelerometer. This may provide greater sensitivity in detecting periods of low activity.

Described herein is a system for treating an obese patient. The system includes an implantable device that has at least one sensor. The device collects patient data with the sensor(s) in response to ingestion by the patient and/or in response to exercise by the patient when the device is implanted in a patient body. A processor is coupled to the implanted device to analyze the patient data to determine sensor-based ingestion information and sensor-based exercise information about the patient. A treatment-applicator display is attached to the processor to receive information about the patient and encourage healthy eating and/or exercise behavior by the patient.

In some embodiments, the implantable device stimulates tissues of the body to inhibit unhealthy ingestion into the body in response to patient data. In other embodiments, the device stimulates tissues of the body to trigger a feeling of satiety or nausea in the patient, without reliance on signal or data received from the implanted sensors.

In more embodiments, the accelerometer collects data during various exercise levels of the obese patient. It is calibrated based on the collected data and a linear relationship between an integral of absolute value of the accelerometer output ($IAA_{tot}$) and a metabolic equivalent (METS)-1.

Described herein is a method for monitoring the energy expenditure of a living body. The method includes coupling an accelerometer to the body to generate accelerometer signals in response to body movement. The accelerometer signals are collected during various body movements and calibrated based on a linear relationship between an integral of absolute value of accelerometer output ($IAA_{tot}$) and a metabolic equivalent (METS)-1. A heart rate sensor is coupled to the body to generate heart rate signals in response to the body's heart rate. A core body temperature sensor is attached to the body to generate temperature signals in response to the body's core temperature. The overall caloric expenditure of the body for a time span using the signals is calculated so that, for a plurality of time portions of the span, the associated energy expenditure rates are identified from the signals. The calculated overall energy expenditure can be displayed or the body can be treated in response to the calculated overall energy expenditure.

In some embodiments, the accelerometer signals are sampled using a first rate and a second rate. Sampling is increased from the first rate to the second rate when the accelerometer output exceeds a predetermined threshold.

Described herein is a device for monitoring a living body. The device includes numerous sensors disposed within the body. The sensors emit signals correlating to the body's energy expenditure. The caloric expenditure of the body is calculated during a time span in response to the signals. The caloric expenditure calculation can be displayed to the body while the body contains the sensors.

Described herein is an apparatus for monitoring a living body. The apparatus includes a power source and a plurality of implantable sensors attached to the power source. The sensors include an accelerometer, a heart rate sensor and a body temperature sensor. The body temperature sensor is implanted in the body's stomach cavity. A processor calculates a caloric expenditure and an ingestion event of the body during a time span in response to the signals. A stimulator is positioned in electrical contact with the stomach. The stimulator is connected to the processor. The apparatus also includes a display coupled with the sensors and the processor.

Described herein is a system for monitoring the health of a living body. The system for monitoring the health of a living body comprises a general wellness diagnostics based on accelerometer, HR and CBT and factors such as duration and quality of sleep, emotional state/stress level/metabolic level, presence of fever, and metabolism.

BRIEF DESCRIPTION OF THE DRAWINGS

Fig. 1 schematically illustrates an embodiment of a stimulation system and sensors.

Fig. 2 schematically illustrates an embodiment of a treatment system.

Figs. 3A-3D illustrates treatment methods.

Figs. 4A-4B illustrate communication methods.

Fig. 4C shows a sample display of a patient's calories in versus calories out during a 24 hour period.

Fig. 5 illustrates a plot showing a linear relationship between $IAA_{tot}$ (accelerometer data) and METS-1.

Fig. 6A shows activity intensity estimated from $IAA_{tot}$ (accelerometer data) during a 24 hour period.

Fig. 6B shows activity intensity calculated using accelerometer and heart rate data during a 24 hour period.

Fig. 6C shows the error in METS between estimated and actual trend lines.

Fig. 7 illustrates a branched algorithm to determine energy expenditure from 1D-accelerometer based activity and heart rate data.

Fig. 8 illustrates a modified branched algorithm using heart rate and 3D accelerometer data.

Fig. 9A illustrates a first configuration of a core temperature measurement concept.

Fig. 9B illustrates an alternative configuration of a core temperature measurement concept.

Fig. 9C illustrates a second alternative configuration of a core temperature measurement concept.

Fig. 9D illustrates a third alternative configuration of a core temperature measurement concept.

Fig. 10 illustrates how core body temperature could be incorporated into a branched algorithm.

Fig. 11 is a flow diagram for detecting a sleep state.

Fig. 12A schematically illustrates a model of core body temperature and heat-flux.

Fig. 12B schematically shows potential locations for a heat flux sensor and temperature sensor.

Fig. 13A illustrates a temperature profile measured along a path from body core to skin surface during rest and exertion.

Fig. 13B illustrates a heat-flux sensor.

Fig. 14 illustrates changes in heat-flux as the distance from the body core changes.

Fig. 15 shows one graphical representation of activity information for a patient that may be provided using the systems and methods described herein.

DETAILED DESCRIPTION OF THE INVENTION

The methods, systems and devices described herein offer improvements over techniques currently used to monitor activity based energy expenditure or treat obesity. The present disclosure relates to monitoring the health and energy expenditure of a living body, treating eating disorders or treating obese patients using implanted sensor-based information so as to affect behavior modification. Although some embodiments of the disclosure make specific reference to treatment for obesity, the methods, systems and devices described herein may be applicable to any treatment in which presenting feedback regarding patients' eating and/or exercise habits is desired.

**Stimulator and Sensor System:**

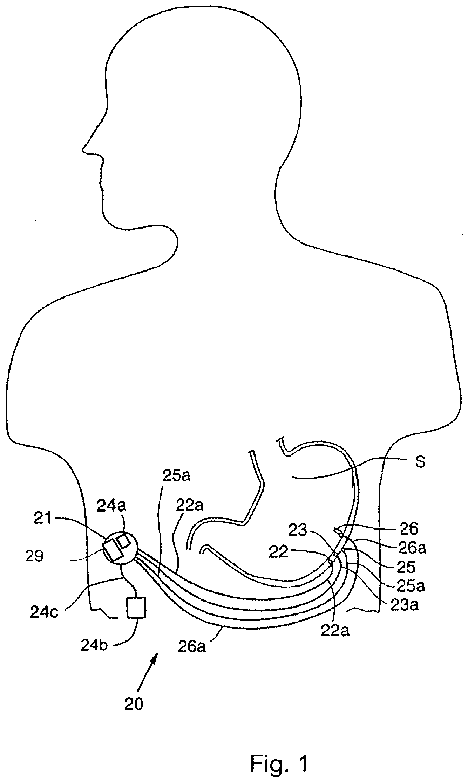

Fig. 1 schematically illustrates a system including a stimulator 20 having an implantable pulse generator (IPG) 21 or implantable device housing (CAN) implanted subcutaneously within a living body. The stimulator further comprises leads 22a and 23a extending from the IPG 21 through the abdomen and to the stomach S where electrodes 22 and 23 are implanted into the stomach muscle layer from the outside of the stomach S. The IPG 21 further comprises a sensor 24a located on the IPG 21 and/or a sensor 24b desirably separate from the IPG and located elsewhere in the patient and coupled to the electronic circuitry 29 in the IPG by lead 24c. The stimulator also includes sensors 25 and 26, that are implanted on or in the stomach S, respectively, with leads 25a and 26a extending from the sensors 25 and 26 to the IPG 21. Sensor 26 is exposed to the inside of the stomach S while sensor 25 is attached to the outside of the stomach. Leads 22a, 23a, 24c, 25a and 26a are electrically coupled to the electronic circuitry 29 located in the CAN/IPG 21.
The exemplary sensors include a core body temperature sensor for sensing temperature information. The potential for using temperature measurements to classify ingestion events is disclosed in Provisional U.S. Patent Application Ser. No. 61/166,636 filed on April 3, 2009.
The sensors may be located on or extend from the IPG and/or the sensors may be located on or extend from a lead or other device. Alternatively or additionally, a sensor may be located separately on the stomach wall and/or a sensor may be otherwise positioned elsewhere within, coupled to or in communication with the patient. The sensors can be implanted for long term use of a month or more to generate signals correlating to energy expenditure of the body.
Activity diagnostics are of high value to a clinician managing an obese patient. Of particular interest is an estimate of energy expenditure which could be measured against caloric intake in order to determine if a patient is in energy balance, energy balance being the state in which energy expenditure is at least as great as caloric intake. Total energy expenditure is a combination of resting energy expenditure, food thermogenesis and activity based energy expenditure.
The overall caloric energy expenditure of the body for the time span is calculated using the signals so that the calculated overall energy expenditure correlates to the first energy expenditure during the first time portion combined with the second energy expenditure during the second time portion. The calculated overall energy expenditure can be displayed and/or the body can be treated in response to the calculated overall energy expenditure.

Activity based energy expenditure is expressed in terms of kilojoules or kilocalories. Energy expenditure is generally the intensity of the activity or exercise multiplied by the duration that the subject is at this intensity. The primary clinical parameter used to describe this intensity may be metabolic equivalents (METS). The advantage of using METS is that it is normalized by the subject's resting energy expenditure, so that 1 MET-hour equals the energy expenditure of that person over one hour of rest. Therefore, using METS allows one to take into account each individual's fitness level. If one is able to determine METS value for every activity of a patient, then the energy expenditure could be calculated by multiplying the METS value for that activity times the duration of that activity. The recommended exercise to maintain energy balance may optionally be expressed in terms of METS. At least 150 minutes at moderate activity or greater (> 3 METS) may be desired for each day for many patients.

**Treatment System:**

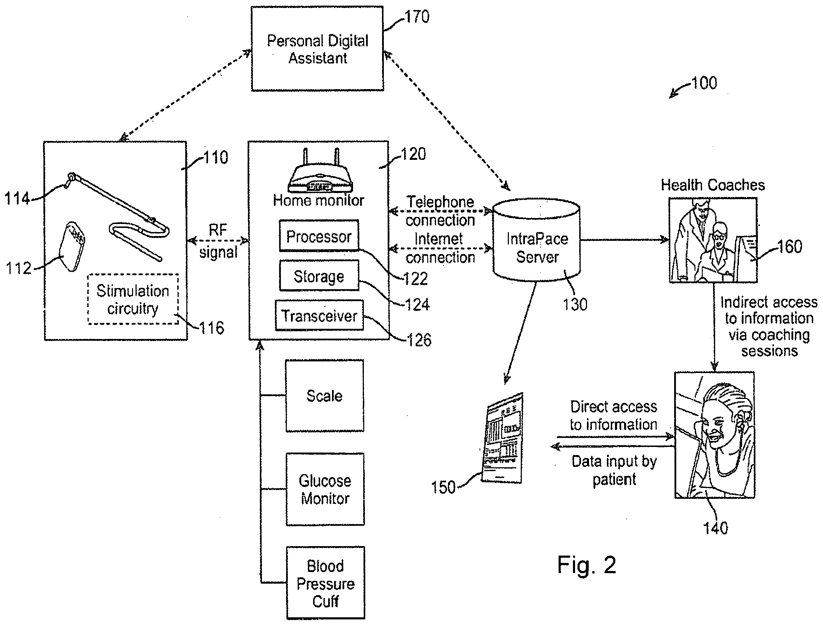

An example system 100 suitable for implementation in embodiments of the present invention is schematically illustrated in Fig. 2. System 100 comprises an implanted device 110 that communicates via a wireless transmitter 112, such as an RF telemetry module. The implanted device 110 includes at least one sensor 114 and, optionally, stimulation circuitry 116 for providing therapeutic stimulation to the patient. A server 130 communicates with home monitor 120 via an internet or other telecommunication system so as to allow access to sensor-based data via a portal 150 and/or health coach workstation 160, thereby providing sensor-based feedback to a patient 140 (through direct presentation or display of the sensor-based information to the patient, and/or through a health-coach/patient relationship).

Each of implanted device 110, home monitor 120, server 130, health coach workstation 160, and portable patient device 170 will typically include associated data processing systems, with the overall feedback system 100 combining their data manipulation and communication capabilities into an overall data architecture. Generally, the data processing systems included in the discreet devices of the invention may include at least one processor. For implantable device 110, this will typically include circuitry implanted in the patient. Other devices of system 100 will include circuitry external of the patient. Such external processor circuitry may include one or more proprietary processor boards, and/or may make use of a general purpose desktop computer, notebook computer, handheld computer, smart phone, or the like. Further details regarding the hardware and software are disclosed in U.S. Patent Application Ser. No. 61/166,636 filed on April 3, 2009.

Sensor 114 in Fig. 2 is coupled to the stomach so as to generate signals responsive to ingestion, with the sensor ideally comprising at least one temperature sensor for sensing temperature information from within the stomach. The sensors may be located on or extend from a housing of implanted device 110 and/or the sensors may be located on or extend from a lead or other device. Alternatively or additionally, a sensor may be located separately on the stomach wall and/or a sensor may be otherwise positioned elsewhere within, coupled to or in communication with the patient. Additional sensors may be included, including an accelerometer and/or a heart rate sensor to measure patient activity or the like. The housing of implanted device 110 will typically contain a battery and circuitry of the implanted device, and may be similar to other known implantable stimulator housing structures used for heart pacemaker systems and the like. A suitable heart rate sensor may comprise an electrode or other sensor engaging the stomach wall so as to receive far field electric signals from the heart. Optionally, such a heart rate sensor may employ the same electrode as used to stimulate stomach tissue to inhibit ingestion, though separate electrodes may alternatively be used. Electrical, acoustic, or pressure heart signals, accelerometer signals, and/or other activity sensor signals may, like temperature or other ingestion sensor signals, be processed and recorded using circuitry 116. Suitable sensors and implantable devices, as well as aspects of the other devices of system 100, may be described in (and/or may be modified from those described in) U.S. Patent Application Ser. No. 12/145,430, filed on June 24, 2008 and U.S. Patent Application Ser. No. 10/950,345, filed on September 23, 2004 US). Processing of sensor signals so as to identify or classify ingestion events and/or patient activity level to be communicated by system 100 (which may occur partially or entirely in implanted device 110, home monitor 120, or server 130) may be more fully understood with reference to Provisional U.S. Patent Application No. 61/122,315, filed on December 12, 2008.

The server 130 contains a number of algorithms designed to evaluate the implanted device data logs in comparison with goals established by the patient and his or her health coaches 160. Based upon the results of the analysis, i.e. whether the goals have been met, coaching messages may be sent to the patient. Specific examples regarding energy expenditure and sleep data include sending encouraging messages for meeting daily or weekly activity goals or sending patient alerts if extended periods of sedentary activity have occurred and/or sleep goals have not been met. With regard to caloric intake, examples include communicating feedback to the patient as to whether eating patterns show adherence to the eating plan or whether caloric intake is meeting daily, weekly, or monthly goals.

Both external and implanted memory of the devices of system 100 will often be used to store, in a tangible storage media, machine readable instructions or programming in the form of a computer executable code embodying instructions and/or data for implementing the steps described herein. The functions and methods described herein may be implemented with a wide variety of hardware, software, firmware, and/or the like as described in (and/or modified from those described in) Provisional U.S. Patent Application Ser. No. 61/166,636 filed on April 3, 2009. Hence, the data processing functionality described herein (and/or the data manipulation method steps described herein) may be implemented largely or entirely within the implanted components, external to the patient, and locally, or remotely, though they may more commonly be distributed at least in part among some or all of the implanted, local, and/or remote data processing components.

As schematically depicted in Fig. 2, aspects of social networking systems 140, 150, 160, with sensor-based information that has been generated using signals from an implanted sensor may be made available to one or more members of a group. Such systems are disclosed in (and/or modified from those described in) Provisional U.S. Patent Application Ser. No. 61/166,636 filed on April 3, 2009.

**Treatment Methods:**

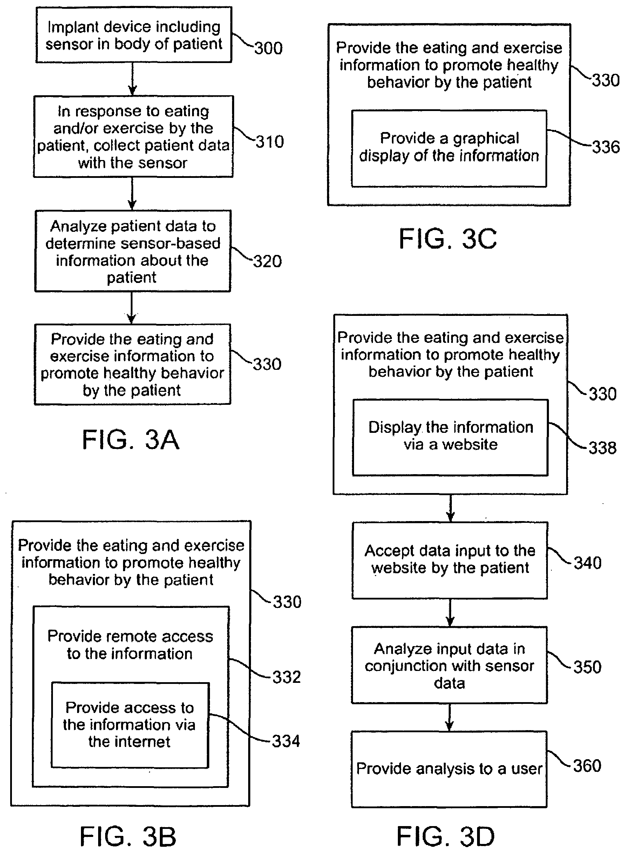

Fig. 3A illustrates a treatment method according to an embodiment of the present invention. Initially, a device including a sensor is implanted in the body of a patient 300. The device may be implanted in the stomach of the patient. Patient data is collected with the sensor in response to an ingestion event by the patient 310. The patient data is then analyzed to determine sensor-based information about the patient 320. The sensor-based information is provided to a user to promote the healthy behavior of the patient 330.

As shown in Figs. 3B and 3C, step 330 may include providing remote access to the information 332, which may also include providing access to the information via the internet 334 and step 330 may include presenting a graphical display of the information 336. In some embodiments, such as illustrated in Fig. 3D, step 330 includes displaying the information via a website 338 and the method further includes accepting data input to the website by the patient 340 and analyzing the input data in conjunction with the sensor data 350. The resulting analysis is then provided to a user 360.

**Communication Methods:**

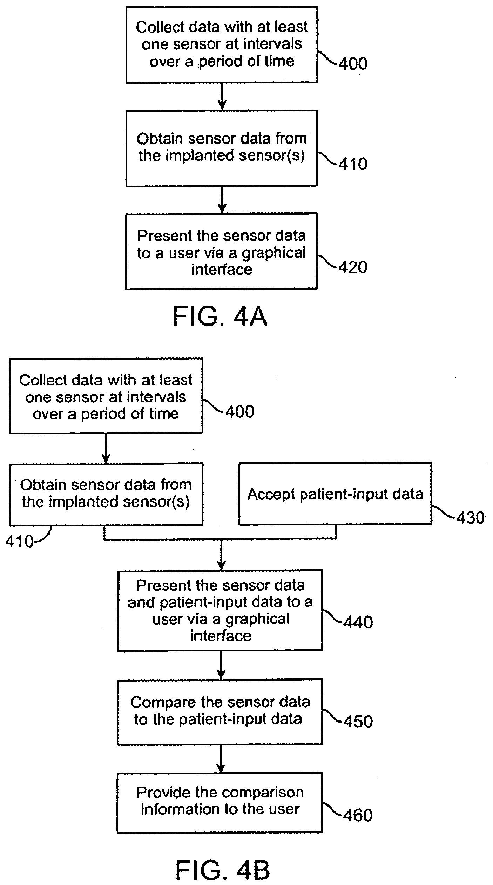

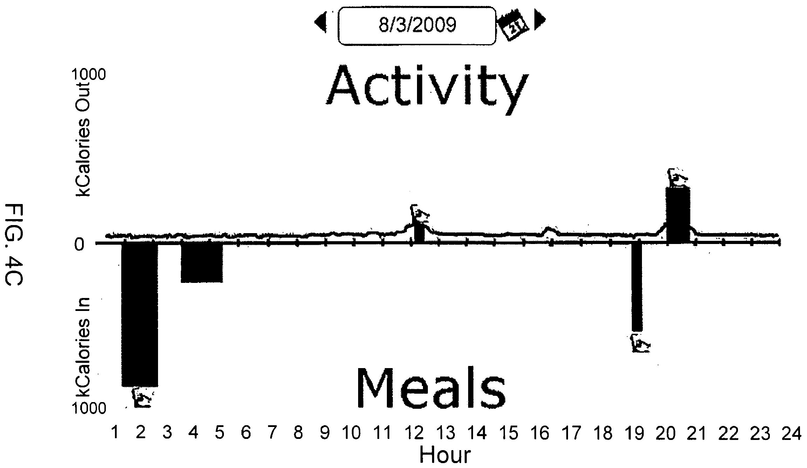

Fig. 4A illustrates a communication method according to an embodiment of the present invention. Data is collected by at least one implanted sensor at intervals over a period of time 400. The sensor data is obtained from the sensor(s) 410 and presented to a user via a graphical interface 420. Referring to Fig. 4B, the method may include accepting patient-input data 430 and presenting both the sensor data and the patient-input data together 440. The sensor data and the patient-input data may also be compared 450 and the comparison information provided to the user 460. The sensor data may include ingestion and/or activity level information as further disclosed in U.S. Patent Application Ser. No. 61/166,636 filed on April 3, 2009. Fig. 4C shows a sample display of a patient's caloric intake versus caloric output during a 24 hour period.

**Sensors and Applications for the Sensor-Derived Measurements:**

Accelerometer and Energy Expenditure due to Activity:

Triaxial accelerometer data can be processed to estimate energy expenditure due to activity. The processing may include the summation of the time integrals of the moduli of accelerometer output from the three separate measurement directions (x, y and z) ($IAA_{tot}$).

$$IAA_{tot} = \int_{t=t_0}^{t_o+T} |a_x| dt + \int_{t=t_0}^{t_o+T} |a_y| dt + \int_{t=t_0}^{t_o+T} |a_z| dt$$

There is a correlation between energy expenditure from activity ($EE_{act}$) and ($IAA_{tot}$) during sedentary activities and during walking. Using the strong linear relationship between $EE_{act}$ and $IAA_{tot}$, energy expenditure from activity during sedentary activities as well as walking can be estimated. Other processing of the triaxial accelerometer signal that yields a value correlated with energy expenditure includes the combined vector magnitude of all three axes, or the vector magnitude of a subset of the axes, the sum of the coefficient of variation of all three axes, or one or more of the axes.

Heart Rate Sensor and Exercise Intensity:

The sensors, shown in Fig. 1, may include an implanted heart rate sensor. Since oxygen is burned with food to create energy, the rate of oxygen consumption is related to energy expenditure. The energy created by burning 1 liter of oxygen is approximately 5 kCal. Heart rate (HR) can be used to estimate energy expenditure since a linear relationship exists between heart rate and oxygen consumption ($VO_2$) during exercise; however there is a non-linear relationship between heart rate and $VO_2$ across active and inactive periods. Heart rate alone can provide a good group estimate, but individual estimates may be quite variable. The combination of heart rate and accelerometer, however, may improve the estimate of energy expenditure.

Heart rate provides a much better measure of exercise intensity than an accelerometer for activities that produce low trunk motion such as bicycle riding and weight lifting, for example. It should also help provide a better activity estimate when additional work is involved while the subject is moving at the same rate such as going up stairs versus going down stairs, running or walking uphill, or moving on an inclined treadmill, for example. Determining maximum heart rate during exercise is also an important parameter for monitoring the fitness level of a patient. In addition, the maximum heart rate during exercise can be used as a threshold for determining if the exercise is at an intensity level that would lead to an increase in metabolic rate, even for a period of time after exercise. The threshold for this exercise intensity may be, for example, 75% of the maximum heart rate, where maximum heart rate can be calculated based on patient age, weight, or entered by a health care provider.

Core Body Temperature Sensor and Heat Flux:

The body uses energy stored in the chemical bonds of food to perform its living functions and work. This metabolic process is inefficient and a significant fraction of the food's chemical energy is converted into internal body heat. The core body temperature may be measured using a thermistor or thermocouple attached to the implanted device housing (CAN) or IPG 21.

The core body temperature can provide a measure of metabolic rate. This metabolic rate changes with activity state and food intake, and follows a daily cycle based on sleep and awake patterns. Food induced thermogenesis may provide about 10% of the average person's daily energy expenditure. A person's metabolic rate plays a large part in their total daily energy expenditure, including the resting metabolic rate which can change with health and fitness level.

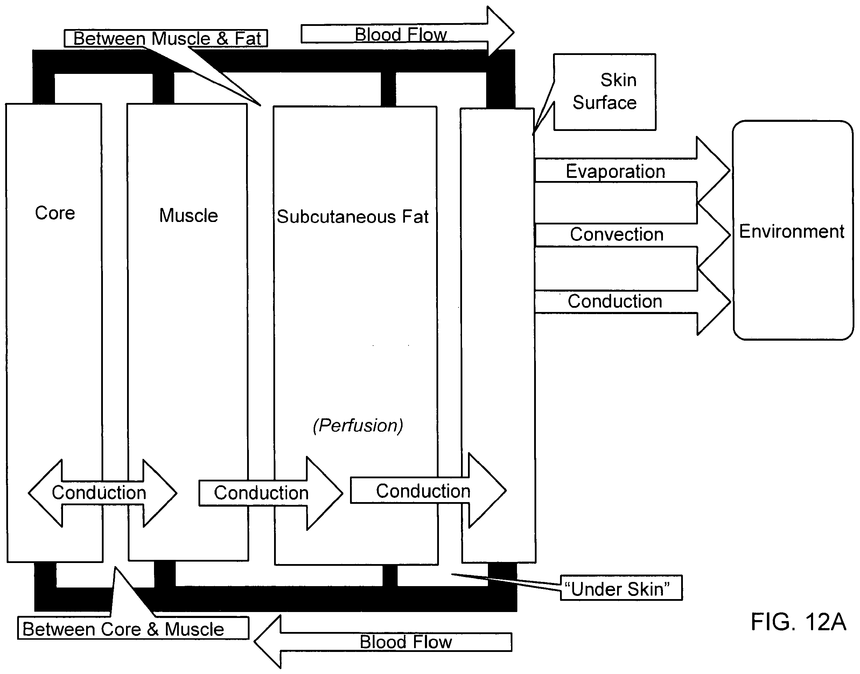

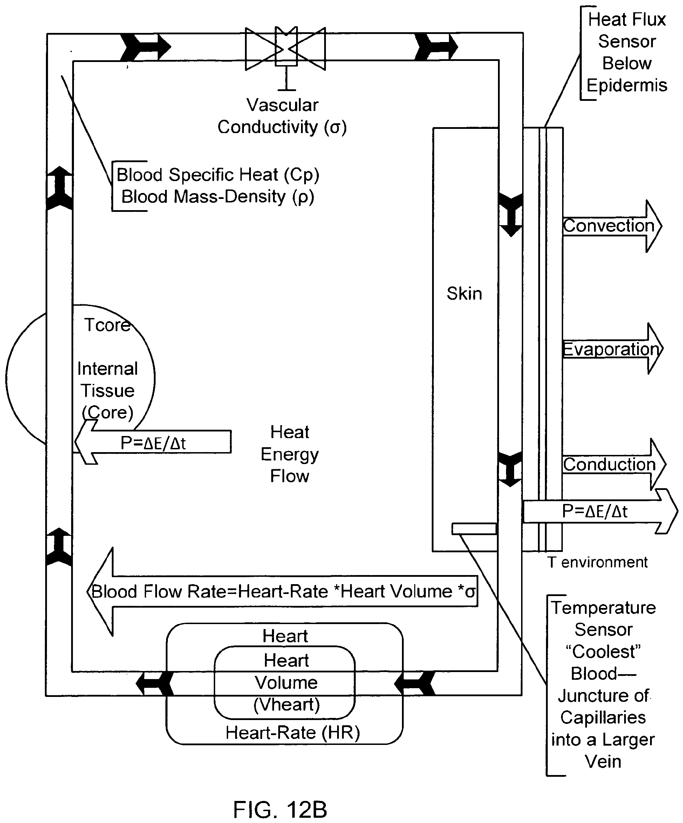

Figs. 12A and 12B show a simple thermal body model of core body temperature and heat-flux. As the body attempts to maintain its core temperature of 37°C, it eliminates excess heat energy. The heat energy flowing from in or near the body core to the external environment (heat-flux), has been used, in part, to estimate the instantaneous total energy being expended by the body. The measurement of this heat-flux from inside the body is complicated by the various means and pathways by which the heat energy is transported and finally transferred to the external environment. The temperature of the body core will change with various activities and exertion intensities and, therefore, may also be considered as a factor in the determination of energy expenditure. The measurement of core body temperature is straightforward, particularly when an implanted temperature sensor is placed in relatively continuous physical contact with core body tissues. Thus, measuring core body temperature may be a suitable substitute for measuring internal heat-flux and may be superior when attempting to determine body energy expenditure in part from the measurement.

The heat flux, shown schematically in Figs. 12A and 12B, may be observed and measured as the body eliminates excess heat to maintain its core and tissue temperature. Heat will flow from the higher temperature core, muscle, etc. to the cooler environment. The body regulates this flow so as to maintain its homeostatic temperature at near 37°C. Some heat energy flows through muscle, fat, and skin by conduction. However, the subcutaneous fat layer is a relatively poor thermal conductor which significantly limits such heat energy flow. Most excess heat from the core and muscle is transported by blood flow to near the surface of the skin where it can then be relatively efficiently transferred to the environment via conduction, convection, and sweat evaporation. The heat flowing from the skin surface to the environment cools the blood and skin. The cooled blood recalculates through the body and again absorbs heat, re-warms, and flows to the skin for subsequent heat energy elimination.

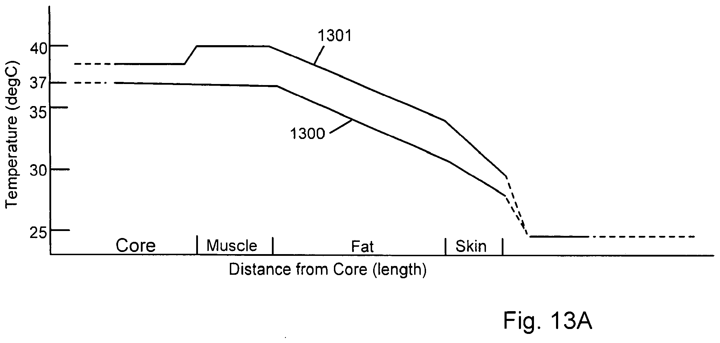

The temperature profile shown in Fig. 13A illustrates what may be observed and measured along a path from the core to the skin surface. The resting profile 1300 shows the core and nearby muscle at approximately 37°C. The temperature decreases through the fat and skin layers to the external environment temperature, for example 25°C. When the body is engaged in additional activity beyond resting, more heat is generated in the muscle and core and the temperature of these tissues increases. This is illustrated by the exertion temperature profile 1301. The temperature of the muscle is here shown to increase more than the core, as it is assumed that the muscle is performing more work; however, this may not always be the case.

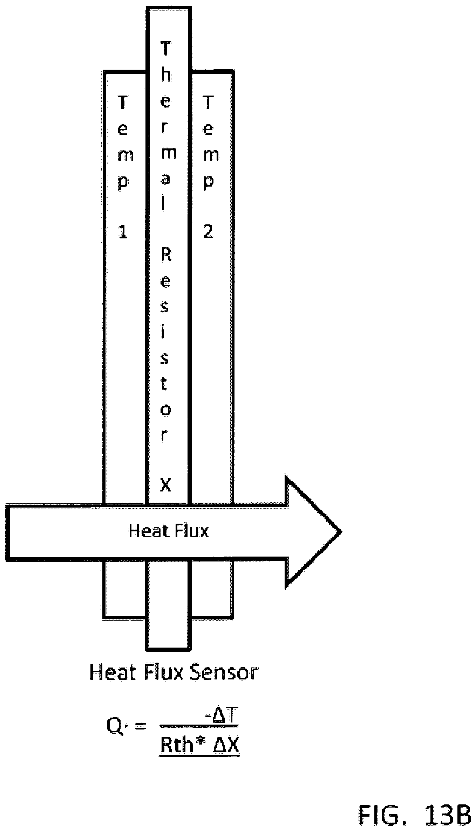

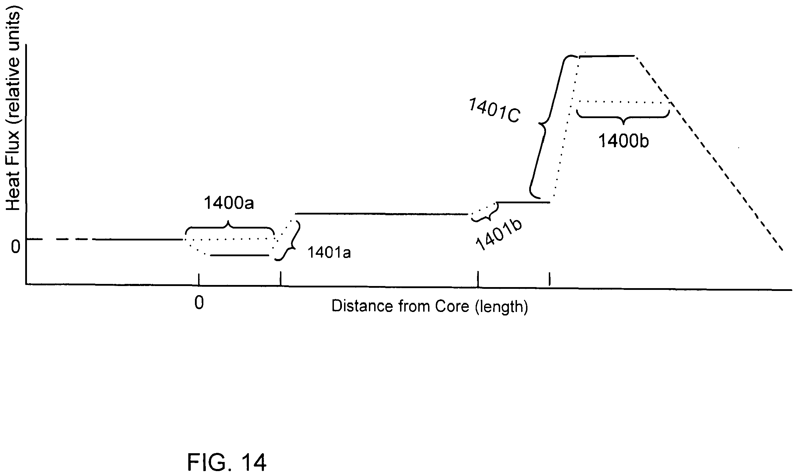

The sloping temperature profile defines a temperature gradient ($\Delta$Temperature/$\Delta$ length), which when divided by the thermal resistivity (Rth) of the tissue yields the heat energy flowing (heat-flux) through the tissue layers. The magnitude of the heat-flux conducted through the various tissues is shown in Fig. 14 for resting 1400a-1400b and exertion 1401a-1401c body states. Note that heat-flux conducted through the body tissues changes relatively little between resting and exertion states. However, heat-flux changes significantly at the skin-surface/environment interface. A simple heat-flux sensor is also illustrated in Fig. 13B. Here, $\Delta$T is the difference in temperature across the sensor, $\Delta$X is the thickness of the sensor, Rth is the thermal resistivity of the sensor's thickness, and Q· is the heat energy per unit time per unit area, or heat-flux (units of power/area) flowing through the sensor. Such a sensor may be located along the thermal path to measure heat-flux through its location.

From this example it can be seen that a change in activity (resting to exertion) will result in both internal temperature and heat-flux increases. The change in internal heat-flux conducted by the tissues is relatively small, more difficult to measure, and may vary with the type of activity and the location of the sensor. The core temperature changes in a similar magnitude to the change in internal conducted heat-flux. However, core temperature is easier to measure, and may more consistently respond to activity type and intensity. Therefore, Core Temperature may be a more suitable and/or consistent factor in determining (total body) energy expenditure than internal conducted heat-flux.

Tilt Sensors and Non-Exercise Activity Thermogenesis (NEAT):

The sensor system may optionally include a tilt sensor for collecting data during various postural allocations (i.e. standing, sitting, laying down) of an obese patient. Some or all of this information may be available from the accelerometer data, which may be used to determine torso orientation to some embodiments from gravitational effects. Non-exercise Activity Thermogenesis (NEAT), a potentially important component in daily energy expenditure, can be evaluated based on this information. The amount of time spent sitting (versus standing), for example, is significantly different when comparing obese versus non-obese subjects. Therefore it may be useful for a physician and patient to be able to evaluate the patient's level of NEAT related energy expenditure, and provide feedback relative to increasing NEAT. For example, a patient could do more activities while standing versus sitting, and do multi-tasking in a way that would add to their NEAT level, (i.e. folding laundry while watching TV), and more ambulatory activities in the work place (i.e. walking to the water cooler, or the copy machine).

The tilt sensors can be used with an implanted system; for example, one tilt sensor could be placed on the device at the hip and a Micro-Electro-Mechanical Sensor (MEMS) tilt sensor implanted in the thigh with radio frequency transmission to the controller in the device.

The present invention can optionally differentiate energy expenditure during NEAT, versus exercise, and shows how the activity splits between these categories on a daily or hourly basis. This will help a subject understand what kinds of activities are burning the most calories, and how lifestyle adjustments can be made to maximize daily calorie burn. Alternatively, four other sitting detection methods, which may or may not involve a tilt sensor on the thigh, could include:

1) an optical sensor that detects a reduction in the amount of fat and cutaneous tissue overlaying the device during standing (when lower abdomen is stretched) versus sitting (when tissue and fat tends to "pouch out" in the lower abdomen);
2) an optical sensor directed toward the pelvis in order to detect the pelvic tilt that occurs when sitting versus standing;
3) detection of reductions in heart rate of 7-11 beats per minute when comparing sitting versus lying; (these reductions may not be differentiable from other factors that affect heart rate, but may be used in combination with activity detection;) or
4) determination of the characteristics of accelerometer signal that correspond to standing versus sitting while doing daily activity.

With regard to the fourth method, examples of these characteristics include a reduction in the vertical acceleration while sitting, or an increase in variance of accelerometer output on all axes over more sedentary activity. The processing needed to obtain these differentiating characteristics may require increased sampling, and reduced temporal averaging compared to detecting exercise level activity.

Referring once again to Figs. 1 and 2, the exemplary systems described herein may employ individual off-the-shelf components, component that are modified or derived from (or otherwise based on) known structures, new and/or proprietary components, or any combination thereof. Exemplary accelerometers or acceleration sensors that might be employed include those commercially available from Honeywell International Inc. of New Jersey, Bosch Sensortec of California, and/or other Microelectromechanical system (MEMS) suppliers, with one exemplary accelerometer comprising a Bosch bma140 analog triaxial low-g acceleration sensor. Suitable temperature sensors might include a wide variety of thermopiles, thermistors, and/or resistance temperature detectors (RTDs), such as those commercially available from TDK Corp. of New York, General Electric, Tyco Electronics of Pennsylvania, Vishay Electronic GmbH of Germany, Panasonic Corp. of Japan, or others, with one exemplary temperature sensor comprising a GE thermistor, part number BR42KA102M. Still other components which may be employed in exemplary embodiments of the devices and systems described herein might include, for example, an implantable lithium ion battery such as those available commercially from Electrochem Solutions of New York, including GreatBatch Tech battery model number QMR 2487.

**Energy Expenditure Determinations:**

Energy Expenditure in an Implanted Device using a Triaxial Accelerometer:

One embodiment of the present invention, includes a system for the estimation of energy expenditure using a triaxial accelerometer in an implanted device. In an implanted device there may be power limitations that force a lower sampling rate for the triaxial accelerometer. The accelerometer may be sampled at a 6 sec interval or 1/6 Hz. This is suboptimal for detection of acceleration in the 0-3.5 Hz range that is common for human acceleration. Two sampling systems that provide power savings along with an improved resolution for determining acceleration include:

1) Adaptive sampling rate, where the sampling rate is increased when accelerometer counts exceed a threshold. The rate could be increased for example to 6 Hz for the period of time when accelerometer counts are above threshold.
2) Varying sampling rate (i.e. every minute the sampling rate is increased to 6 Hz for a period of 15 seconds). This would yield a total number of samples of 90 per minute, versus 360 samples per minute at a constant rate of 6 Hz.

Group Calibration:

In one embodiment, a group calibration would be performed for the purpose of determining the calibration factor between activity intensity in terms of METS, and accelerometer output (for example $IAA_{tot}$). The group calibration would be preferably done in subjects who have similar characteristics to the patient population in whom the device will be implanted. Alternatively, or in addition to the group calibration, individual calibration could be performed in the clinic at 2-6 week follow-up after device implantation. The individual calibration factor would replace the group calibration factor, leading to a better energy expenditure estimate for the individual.

A group of obese subjects would be asked to perform activities that are known to fall into the different activity classifications listed below, sedentary, light, moderate, and vigorous. These classifications are used when describing physical activity guidelines, and would therefore be useful, and meaningful to a clinician. Desirably these volunteer subjects would have similar characteristics to the patient population that will receive the implanted device.

The intensity of each activity would either be pre-defined with regards to METS, or the METS value would be determined through another measurement technique, either an indirect calorimeter, or another validated energy expenditure determination. A MET can be used as a clinical index that can be defined as the relative intensity of a specific physical activity performed in the steady state, where 1 MET is equivalent to an individual's resting energy expenditure. The estimated activity intensity in terms of METS is given for some common activities in the following table:

| Classification | Intensity | Example Activities |
| --- | --- | --- |
| Sedentary | 1 MET (corresponds to resting EE) | Lying down, TV watching, reading, working at desk |
| Light | <3.0 METS | Washing dishes, folding laundry, household chores, slow walking. |
| Moderate | 3.0 - 6.0 METS | Fast walking |
| Vigorous | > 6.0 METS | Jogging, climbing stairs. |

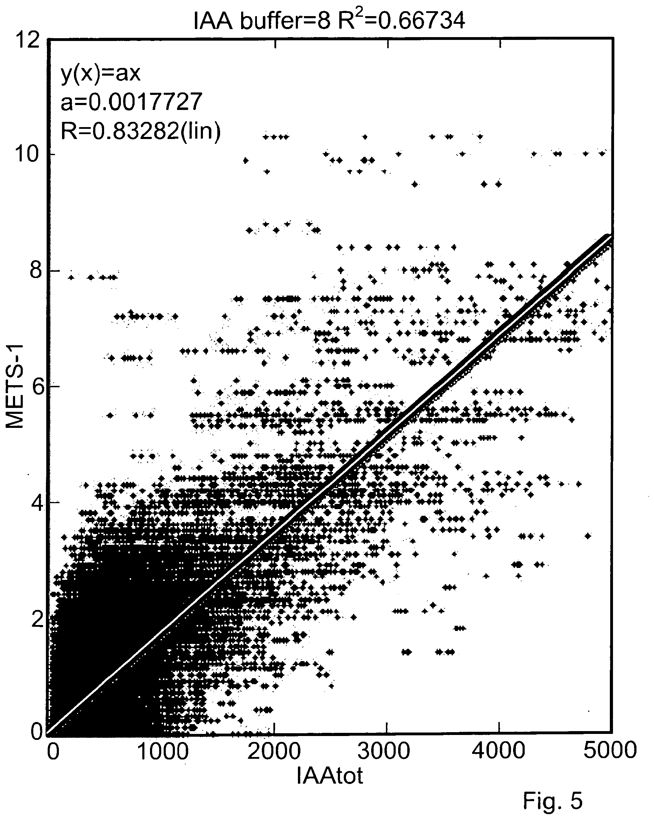

The activity protocol should include periods of time greater than 20 minutes where the subject is at each activity intensity, and optimally include a 24 hour period during which the subject performs normal lifestyle activities. Data from the accelerometer would be obtained during this activity protocol. The calculated $IAA_{tot}$ would be then plotted against the known activity intensity in METS (based on simultaneous measurement in validated device, or output on exercise machine). A linear regression would then be determined from this data, as shown in Figure 5. The plot below shows METS-1 (since METS = 1 is the minimum value for METS, given 1 equals resting energy expenditure) versus $IAA_{tot}$, for a combined data set from 6 subjects. The best linear fit was at: y = 0.017727 x. This means the calculated calibration factor is 0.017727, or METS-1 = 0.017727 * $IAA_{tot}$.

Individual Calibration:

Because each accelerometer may have its own response characteristics, and because the characteristics of an individual's body motion during walking exercise may differ, it would be valuable to do individual calibration of the accelerometer output, $IAA_{tot}$ for each patient relative to METS. It may not be feasible to do a full calibration study for each patient that would include 24 hours of data collection with the implanted device and a validated energy expenditure measurement device. Hence, a shortened protocol may be done in the clinic where the individual would be asked perform activities at known METS levels. In a clinical setting, the clinician could be led through a calibration protocol by an external instrument (such as a computer or other device 160 of a clinician or other health coach), where the start and stop time of particular activities would be triggered by the external instrument with visual or audio clues. The external instrument would upload the appropriate accelerometer values from the device corresponding to the various activities level. Then the programmer could automatically calculate the calibration factor specific for that patient based on the calculated $\text{IAA}_{tot}$ during each activity level.

Post Exercise Metabolism:

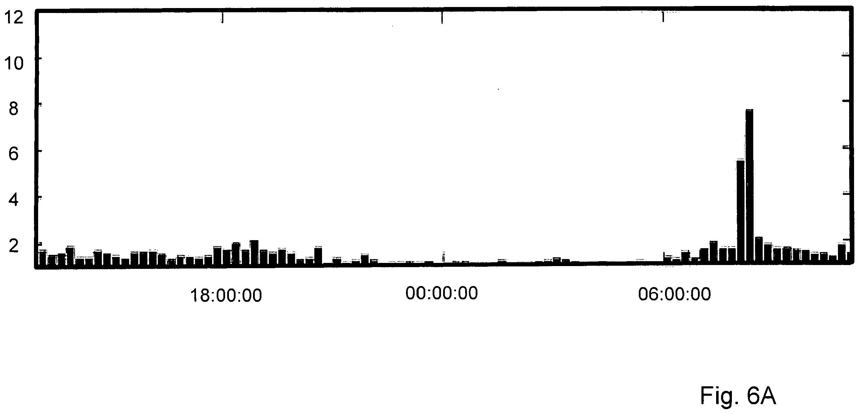

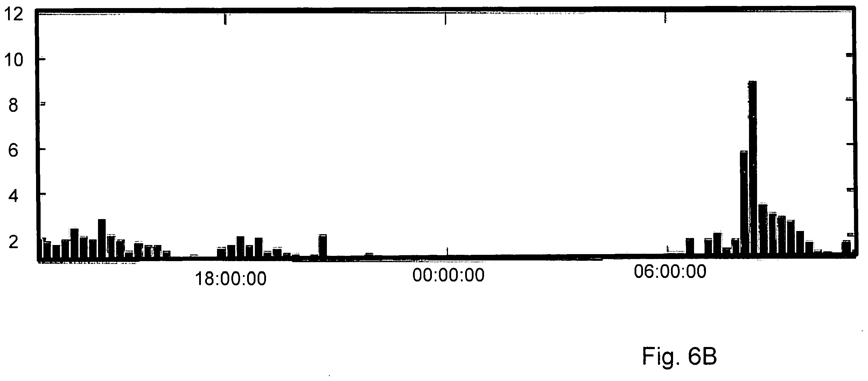

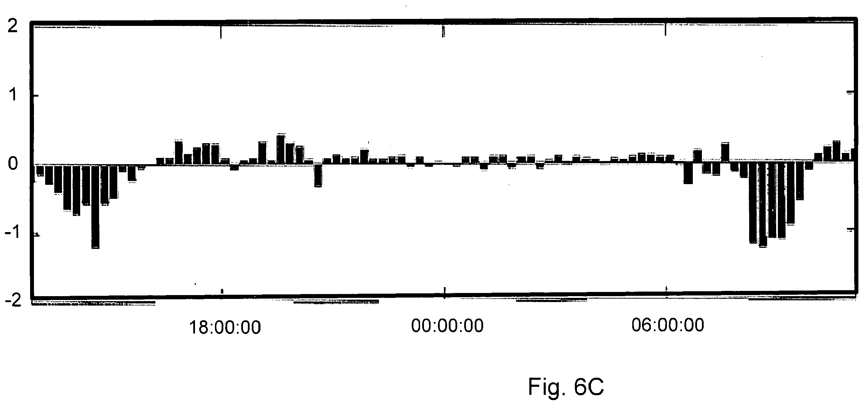

When the intensity of activity goes above a threshold value for a period of time, the metabolism of the person increases for a period of time after exercise. This increased metabolism leads to increased energy expenditure in the post exercise period. Fig. 6A represents the activity intensity estimated from $\text{IAA}_{tot}$ (accelerometer data), the middle plot of the activity intensity calculated based on accelerometer and HR data, and the bottom plot is the error in METS between the estimated and actual trend lines. The accelerometer and HR intensity estimation shown here was determined using an Actiheart® system. The exercise period in this case began at 7:30 AM. The large underestimate of energy expenditure by the accelerometer immediately following the exercise period may be due in part to the fact that an accelerometer based system does not sense increased energy expenditure due to increased metabolism post-exercise.

In the absence of a heart rate measure, the increases in energy expenditure after activity could be modeled from other measured parameters. A decay function could be used that is dependent on the intensity and duration of the workout. For example the average energy expenditure could follow an exponential decay function:

$$N(t) = N_0 e^{-\lambda t}.$$

$$EE(endofexercise + t) = EE(endofexercise)e^{-dt}$$

Where:

d = decay constant that could take the following form:

$$d = (b1 \times I) \times (b2 \times D)$$

where I = the intensity of the exercise, D = duration of the exercise, b1 and b2 are constants,
where b1>b2 because intensity has more effect on the post-exercise metabolism than duration.

Energy Expenditure in an Implanted Device using Triaxial Accelerometer and Heart Rate (HR):

In another embodiment of the present invention, using a triaxial accelerometer sensor (as described above in terms of $\text{IAA}_{tot}$ and a heart rate sensor, provides a more accurate determination of energy expenditure. An implanted system as described herewith could include a wide bipolar or unipolar sense vector in the abdominal cavity. A wide bipolar or unipolar vector will successfully pick up far-field electrical activity such as the electrocardiogram. Possible vectors include the stimulation electrode on the stomach wall to the subcutaneously implanted CAN/IPG, or between the stimulation and reference electrode on the stomach wall. Lead systems and additional details to implement such a system can be found in U.S. Patent Application Ser. No. 12/145,430 filed on June 24, 2008 and U.S. Patent Application Ser. No. 61/166,636 filed on April 3, 2009.

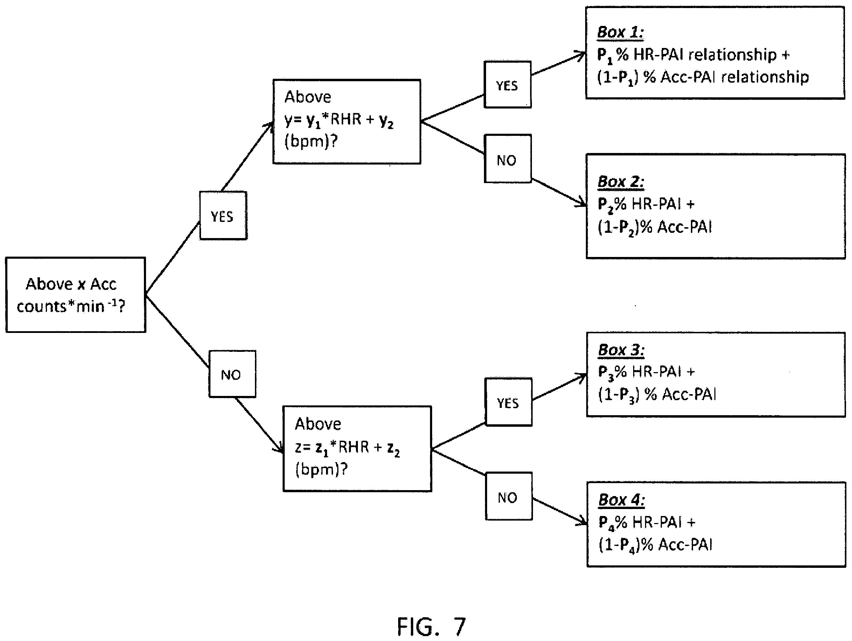

Fig. 7 shows a branched algorithm using heart rate and accelerometer based activity to determine energy expenditure. Additional details regarding this algorithm are described in Brage, S., et al., Branched equation modeling of simultaneous accelerometery and heart rate monitoring improves estimate of directly measured physical activity energy expenditure. J Appl Physiol, 2004. 96:343-351. This algorithm is meant to determine activity based energy expenditure. Two regression equations are used, a quadratic equation that translates heart rate to physical activity intensity (PAI) and a linear one that translates accelerometer output (Acc) to PAI. The purpose of the threshold x for activity is to differentiate periods of higher heart rate that may be due to stress, caffeine, or other non-activity factors, but to include activities such as biking which have a low accelerometer output.

During the highest activity state the heart rate and accelerometer components are combined using factor P1 (Box 1), where P1 is close to 1. This reduces the weight of the accelerometer based component since it is multiplied by (1 - P1), while the heart rate based component is multiplied by P1. Box 2 represents a lower activity state since the heart rate didn't meet the threshold $y = y_1*RHR+y2$ (beats per minute (bpm)); in this case the heart rate and accelerometer components are combined using factor P2, which is less than 0.50, reducing the heart rate contribution to the PAI. Both Boxes 3 and 4 represent states when the accelerometer based activity is low (below the threshold x), but the heart rate threshold (below the $z = z_1*RHR + z1$ bpm) is used to differentiate the cases where activity that the accelerometer may detect is low, but heart rate indicates more activity. When the activity is low (below x), but the heart rate is above threshold, then the factor P3 is used to combine heart rate and accelerometer output to produce PAI (Box 3); when the heart rate and activity are low, P4 is used (Box 4). Both P3 and P4 are below 0.5, reducing the heart rate component of the calculated PAI relative to the accelerometer. In this model $P1 \geq P2 \geq P3 \geq P4$; at lower activity the HR component of PAI is reduced and the accelerometer based component is emphasized. The regression model that produces the PAI based on activity is designed to produce a PAI of zero at activity levels below slow walking. In order to access daily energy expenditure, it is desirable that low level non-exercise activities also be included in the energy expenditure model.

In an embodiment of the present invention, the algorithm shown in Fig. 7 provides greater sensitivity in low activity detection by several methods including:

1) Using a 3D accelerometer provides a more sensitive measure of activity. The calculation of the $IAA_{tot}$ output as described above, produces an output that is related linearly with energy expenditure over a number of daily activities. The $IAA_{tot}$ may provide a more sensitive threshold than Acc. The threshold x may be determined by using the linear regression between $IAA_{tot}$ and METS as described above, and choosing a threshold in the range of 1.0 - 2.0 METS. Unlike a 1D accelerometer, the output of a triaxial accelerometer may be linearly increasing through the walk to run transition, so the y threshold shown on Figure 5 can be avoided if desired.

2) Using an triaxial accelerometer a more precise differentiation between intermittent lifestyle activities and other activity bouts such as walking, running, stair climbing, and lawn-mowing, can be achieved by calculating the coefficient of variation (CV) in the activity counts over 15 second spans and using this CV as a threshold to choose between two regression equations for determining energy expenditure from accelerometer output. This type of algorithm can be used to determine METS from accelerometer output with a threshold x in the range of 1.0-2.0 METS.

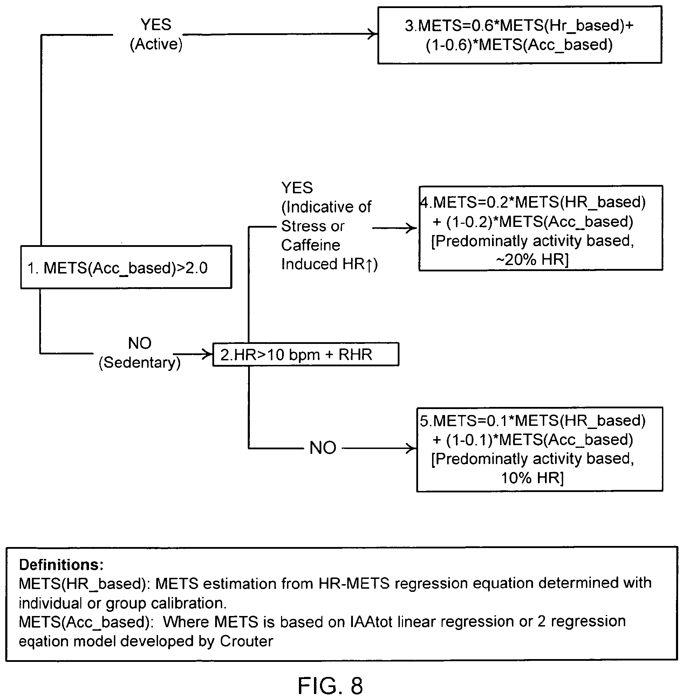

Fig. 8 illustrates a branched algorithm for determining energy expenditure from a triaxial accelerometer and heart rate which utilizes the advantages of the triaxial accelerometer. Block 1 describes the initial activity based threshold that differentiates sedentary activity from daily living activity, walking and other exercise activity. The threshold is optimally between 1.0 and 2.0 METS. If the activity intensity (METS) meets this threshold, then Block 3 describes the equation that will combine the METS determined from the heart rate regression (*METS(HR_based)*) with the METS determined from the accelerometer based regression (*METS(Acc_based)*). As indicated in the definition METS(Acc_based) is a regression relationship that can be described by one or more regression equations. If the subject appears sedentary based on the accelerometer output, then in Block 2 if HR is greater than 10 bpm over resting heart rate (RHR) then Block 4 describes how METS will be determined using an equation that will combine the METS determined from the heart rate regression (*METS(HR_based)*) with the METS determined from the accelerometer based regression (*METS(Acc_based)*). In this case, since the heart rate is significantly above RHR, we can make the assumption that the heart rate is elevated for a reason that is non-activity based and the weight of the heart rate component in the METS calculation is reduced to 20% (or between 0-50%). If the HR and activity are low then Block 5 describes the equation that will combine the METS determined from the heart rate regression (*METS(HR_based)*) with the METS determined from the accelerometer based regression (*METS(Acc_based)*), with the weight of the heart rate-based calculation of METS reduced further to 10% (or between 0-50%).

Energy Expenditure Estimated Using Heart-Rate and Core-Bod-Temperature

Much of the internal body heat generated by a body must be removed (transferred to the environment) in order for the body to maintain its desired internal temperature-nominally 37°C. The body can transport heat to the environment by heat conduction through tissues from the warmer interior to the typically cooler exterior environment, and via blood flow from warmer interior tissues to the typically cooler skin layer. This heat is then transported from the skin layer into the environment via heat conduction to a cooler environment, convection, and/or evaporation - as sweat changes from liquid to gas as it evaporates into the environment. Energy transport via radiation is negligible at nominal body and room temperatures and is ignored here. Heat conduction directly from the interior or core of the body to the environment is significantly limited by the relatively low thermal conductivity of the subcutaneous fat layer. Thus, a majority of internal or core body heat is transported to the skin and subsequently to the environment via blood flow. The rate of heat energy transported from the body core to the environment via blood flow may be used as a factor in the estimation of total body energy expenditure.

Rate of Heat Energy Transported by Blood Flow

Heat energy transported by blood flow may be measured directly using a heat flux sensor. BodyMedia of Pennsylvania offers a heat flux sensor which can be placed externally on the human body. The data collected by an externally-placed heat flux sensor may suffer from inaccuracies due to heat dissipation upon leaving the epidermis, along with other variables such as temperature and other environmental conditions which could affect the data recoding. To enhance accuracy of the readings, the heat flux sensor used to measure the power dissipating through the skin may be located just below the epidermis. Fig. 12A shows a potential location for the heat flux sensor. Implanting the sensor near or immediately below the epidermis would place it directly in the path of the expended energy leaving the body (as heat through the skin) avoiding the challenges of accounting for external temperature and sweat generation and evaporation. The implantation of the heat flux sensor under the epidermis can also facilitate an interface with and/or connections to internal and external components of the IntraPace System.

When a direct measurement of heat energy by blood flow is not available or practical, this value may be approximated. We define $P_{rest}$ as the average heat energy per unit time (power) transported by blood flow from the core of the body to the environment when the body is at rest. $T_{37}$ is defined as the core body temperature, assumed to be 37°C, and $T_{env}$, the effective temperature of the environment.

The value of $P_{rest}$ may be approximated as follows.

The specific heat of a material, e.g., blood, ($c_p$) at constant pressure is the ratio of the amount of (heat) energy ($\Delta E$) required to change the temperature ($\Delta T$) of mass (M).

$$So, c_p = \Delta E / \Delta T / M, \text{ or } \Delta E = c_p \Delta T \ M.$$

Let the material be blood. Then the mass term (M) is a product of the blood's mass-density (p) and its volume (V) or $\rho V$.

So, $\Delta E = c_p \Delta T \rho V$. Then, per unit time $\Delta t$, $\Delta E/\Delta t = c_p \Delta t \rho (V/\Delta t)$.

Now, $\Delta E/\Delta t = P$ ; P is power, defined as energy per unit time.

$$So, P = c_p \Delta T \rho (V/\Delta t)$$

($V/\Delta t$) is the blood flow rate (volume per unit time). Blood flows/circulates from the heart, through arteries, capillaries, and veins, and returns to the heart. It is warmed as it passes through the internal or core tissues and cooled as it flows near to surface of the skin. The blood flow rate through this circuit is the product of the stroke volume ($V_{heart}$), the heart rate (HR), and a vascular conductivity factor ($\sigma$).

Thus,

$$V/\Delta t = V_{heart} \ HR\sigma. \ So, P = c_p \Delta T \rho \ V_{heart} \ HR\sigma.$$

From here we can approach the problem a couple of ways:

Method 1: Determine the power the body must transport from the core to the environment to cool the body to the normal body temperature 37°C, and equate that to energy expenditure. Assume that vascular conductivity, stroke volume, and temperature of the environment are constant between rest and activity. Assume that power is correlated with energy expenditure using a proportionality constant that comprises other "constants" including stroke volume, vascular conductivity, and the specific heat of blood. Use a known method to measure energy expenditure, heart rate and body temperature at rest and activity. The two equations for energy expenditure at rest and activity can then be used to solve for the proportionality constant, and the effective temperature of the environment ($T_{env}$). The 'effective temperature of the environment' term ($T_{env}$) is really an approximation for the coolest temperature of the blood as it leaves the skin. This could be measured by a temperature sensor located at or just below the skin surface. A similar argument to making the $\sigma$ term a constant can be made as for the $T_{env}$ term.

Fig. 12B schematically shows a potential location for at least one temperature sensor. Desirably $T_{env}$ will be

measured just below the skin layer, where the blood returning from the heart reaches its lowest temperature. Such measurement may be difficult to implement in practice because the blood flow is dispersed in fine capillaries within the skin - so that heat transfer is improved. A very small temperature sensor could be implanted within the body at a location where the capillaries coalesce rapidly into a larger vessel. A temperature sensor for $T_{env}$ positioned at such a location may provide a more accurate and dynamic estimate of $T_{env}$ which would be more accurate than assuming a constant value for $T_{env}$.

Alternatively, a temperature sensor located just below the epidermis layer of the skin would also provide a reasonable estimate of $T_{env}$. However, the measured temperature at this location may be more subject to errors introduced by the temperature of the air immediately above the skin. A significant external temperature change, such as an impinging cold wind, could then result in an erroneous temperature measurement.

$\Delta T$ is defined as the difference between the core body temperature ($T_{37}$) and the temperature of the cooler blood returning from the skin layer, which is approximated by an effective temperature ($T_{env}$); thus $\Delta T = (T_{37} - T_{env})$.

Then,

$$P_{rest} = c_p (T_{37} - T_{env}) \, \rho \, V_{heart} \, HR_{rest} \sigma.$$

Although $\sigma$ and $V_{heart}$ may not be constants, they will be considered as such for the remainder of this analysis (see comment below).

Let

$$A = c_p \, \rho \, V_{heart} \, \sigma, \ \text{then } P_{rest} = A \, HR_{rest} \, (T_{37} - T_{env}).$$

This is approximately the amount of power or energy per time, e.g., in Kcal/hr or Watts, that the body at rest transports to the environment to cool (or heat) the core, depending on the $T_{env}$, to maintain the body at $T_{37}$.

When the body performs work e.g., exertion or exercise, or experiences stress or illness, additional internal heat will be generated. Some of this additional internal heat will cause the core body temperature to increase to $T_{act}$. The body will then increase the heart rate to $HR_{act}$, which increases the blood flow rate, to remove this additional heat as the body attempts to maintain its core body temperature at 37°C. Pact is defined as the power transported by blood flow in this active state and can similarly be approximated by:

$$P_{act} = A \, HR_{act} \, (T_{act} - T_{env}),$$

or more generally as METS=P(t,HR)/Kg.

Estimation of Energy Expenditure from Heart-Rate and Core-Body-Temperature

At the two body states (rest and active) heart rates and core body temperatures can be directly measured. In addition, Energy Expenditures may be estimated/measured by various means e.g., LifeCheck. Then by determining EE, HR and T at two states, C and $T_{env}$ can be calculated (when assumed constant). Then the expression

$$(METS*Kg) = A* (HR) (T_{core} - T_{env})$$

may be used to estimate energy expenditure from measurements of heart-rate and core-body-temperature.

The limitations of this method are the assumptions that the vascular conductivity and the stroke volume are constant, and also that the $T_{env}$ will not change between rest and activity. In reality, as the body puts off heat during activity the temperature of the environment near the surface of the skin will increase.

Method 2: Determine the Power that was generated to heat and maintain the blood to an elevated temperature during activity as it travels through the internal muscle tissue and equate that to energy expenditure. Make the assumption that the temperature of the environment will not vary greatly in normal device use, and that it will be similar to the environmental temperature during system calibration.

Use the equation derived above, but since we are determining the energy expenditure to heat the blood above a temperature of 37 ° C or $T_{rest}$, $\Delta T = T_{core} - T_{rest}$, so:

$$\text{METS*Kg} = \text{A*(HR)} \, (\text{T}_{core} - \text{T}_{rest})$$

Perform a calibration study where subjects step through increasing activity levels from resting state up to vigorous exercise, while measuring energy expenditure, HR, and core body temperature. The constant C (that includes vascular conductivity, stroke volume) will be solved for at each activity level. From this we can determine C as a function of $\text{T}_{core}$, $\text{C}(\text{T}_{core})$; this may be a linear or exponential relationship or other. This function could be determined on an individual basis in a calibration following device implant, or determined from a group calibration and hard-coded into the device algorithm. Once $\text{C}(\text{T}_{core})$ has been derived, then EE may be calculated based on the equation shown below:

$$\text{METS*Kg} = \text{A}(\text{T}_{core}) * \text{HR*}(\text{T}_{core} - \text{T}_{rest})$$

The environmental temperature will affect the $\text{C}(\text{T}_{core})$ function, and this effect will be taken into account by the calibration study. But the limitation of this method is we are making the assumption that the temperature of the environment will not vary greatly in normal device use, and that it will be similar to the environmental temperature during system calibration.

Energy Expenditure in an Implanted Device using Triaxial Accelerometer and Heart Rate (HR) and Core Body Temperature (CBT):

Figs. 12A and 12B show how the internal body heat produced by the metabolic process during activity is transferred to the environment in order for the body to maintain its desired internal temperature of 37°C. The body can transport heat to the environment by: (1) heat conduction through tissues from the warmer interior to the (typically) cooler exterior environment; and (2) via blood flow from warmer interior tissues to the (typically) cooler skin layer. As shown in Figs. 12A and 12B, this heat is then transported from the skin layer into the environment via (i) heat conduction to a cooler environment, (ii) convection, and/or (iii) evaporation. Energy transport via radiation is negligible at nominal body and room temperatures and is ignored here. Heat conduction directly from the interior (core) of the body to the environment is significantly limited by the relatively low thermal conductivity of the subcutaneous fat layer. Since a majority of core body heat is transported to the skin via blood flow, the rate of heat energy transported from the body core to the environment via blood flow may be used as a factor in the estimation of total body energy expenditure.

As described in the introduction, using the accelerometer and heart rate for energy expenditure determination has limitations. An accelerometer can help determine if the heart rate is high due to stress or caffeine alone, but algorithms are dependent on determining a threshold x (as shown in Figure 7 and previously discussed) that must include activity such as bike riding which is difficult to detect with an accelerometer on the torso, but not include low level activity where an increased HR would not be expected. It can be difficult to determine this threshold with the many different types of activities that are part of a subject's daily activity. Using core body temperature to estimate energy expenditure as described above, would allow better determination of physical activity since work done by muscles, regardless of the location in the body will produce elevation of core body temperature and allow better differentiation between physical activity and other things that raise heart rate such as stress and caffeine.

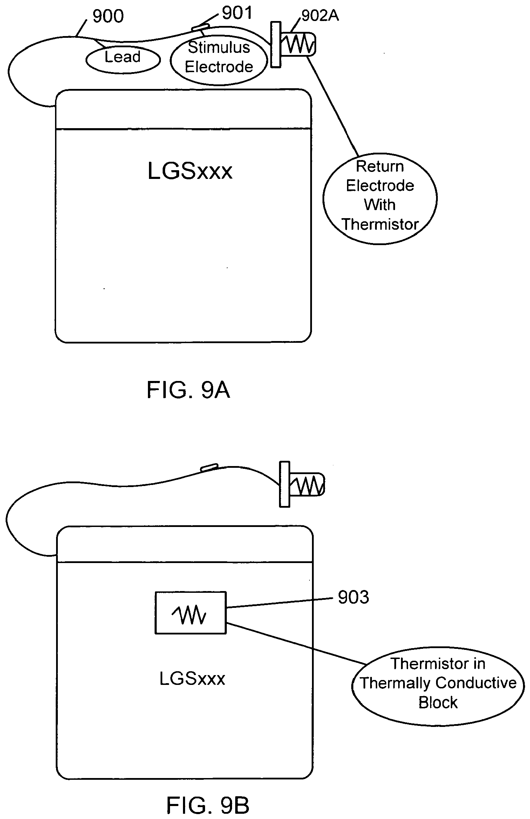

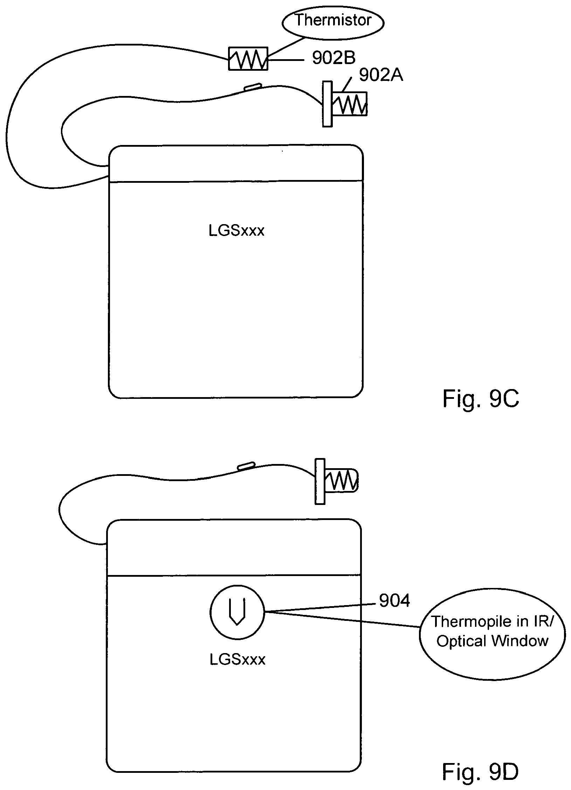

In yet other embodiments of the current invention, Fig. 9A-D illustrates temperature measurement concepts. Fig. 9A includes a lead 900, and return electrode with a thermistor 902A connected by a stimulus electrode 901. An additional internal temperature sensor, Fig, 9B, includes components of Fig. 9A and a thermistor in a thermally conductive block 903. Fig. 9C includes an additional external thermistor 902B. Fig. 9D depicts a thermopile 904 in an internal configuration. Various other alternatives, modifications and equivalents may be used. Therefore, the description above should not be taken to limit the scope of the invention which is widely defined by the appended claims.

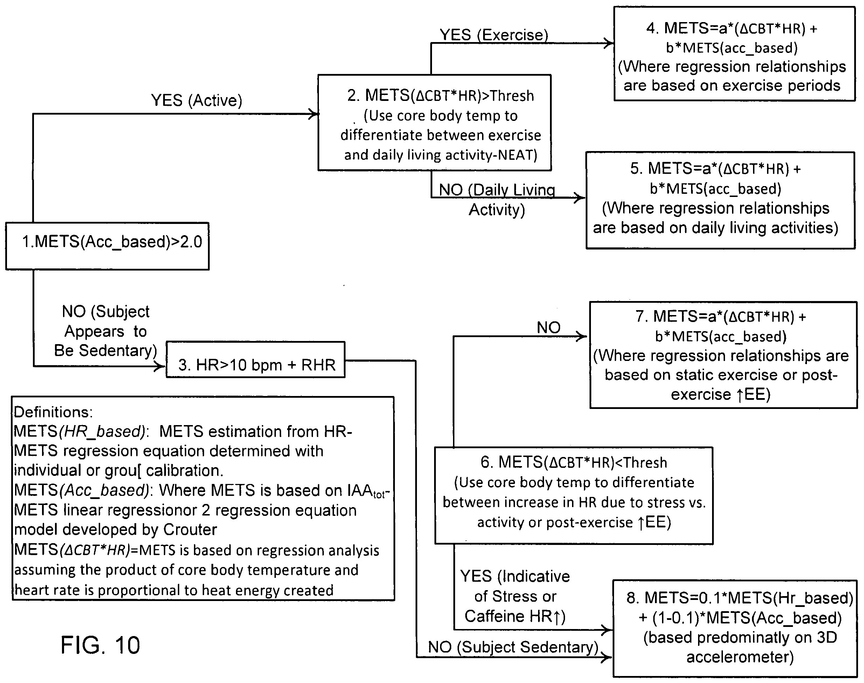

Fig. 10 illustrates an example of how core body temperature could be incorporated into a branched algorithm according to embodiments of the present invention. Block 1 of Fig. 10 is meant to differentiate sedentary activity from daily living activity or exercise activity. This initial differentiation may be based purely on activity. Block 2 will then further differentiate between exercise and activities of daily living (which usually fall between 2.0 and 3.0 METS). This differentiation will be based on the estimated energy expenditure based on core body temperature and heart rate as described above METS(ΔCBT * HR). Blocks 4 and 5 describe the equation that will combine the METS determined from the heart rate and core body temperature regression METS(ΔCBT * HR) with the METS determined from the accelerometer based regression (*METS(Acc_based)),* for exercise and activities of daily living respectively. Block 3 describes a heart rate (HR) based threshold that is used to determine if there is a need to differentiate a higher heart rate due to stress versus activity or a post-exercise increase in metabolism. If the HR is more than 10 bpm above resting, and if estimated energy expenditure based on HR and CBT is higher than a threshold (Block 6) it is assumed the subject is involved in a static work activity or in a post-exercise period, and the activity level estimation is based on the equation in Block 7. If not, it is assumed that the higher heart rate is due to stress of caffeine, so the activity level of the subject will be estimated predominantly based on activity in Block 8.

Fig. 10 shows how different regression relationships between METS and HR * CBT, or METS and accelerometer based activity can be determined for different activity states. This leads to a better estimation of energy expenditure. In the equations described in Blocks 4, 5, 7, and 8, the constants $a$ and $b$ add to up 1, and are used to specify whether the accelerometer based METS estimation, or the HR* CBT based METS estimation will predominate. These factors $a$ and $b$ may be determined empirically through a clinical study where a validated determination of METS is obtained (i.e. through indirect calorimetry), and the coefficients are adjusted and/or optimized to reduce estimation errors. Another embodiment could include the estimation of METS with a multiparameter equation involving all three variables, (i.e. METS = $a$* HR + $b$* CBT + c * $IAA_{tot}$). This equation could be linear, quadratic or biquadratic, for example.

Sleep Detection Using an Implanted Device using Triaxial Accelerometer and Heart Rate (HR) and Core Body Temperature (CBT):

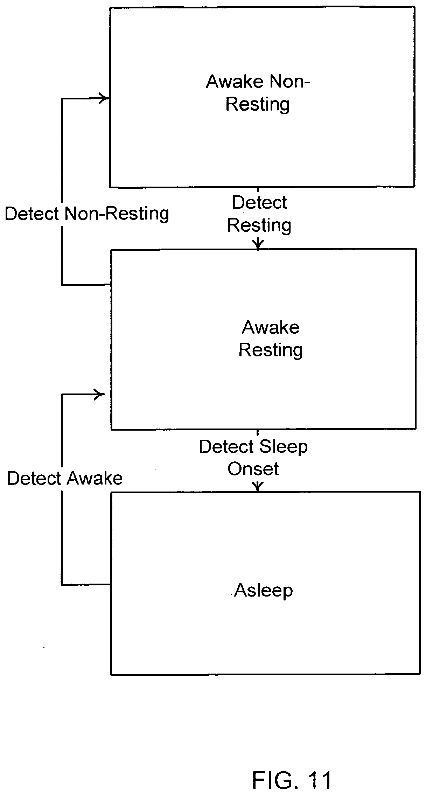

In embodiments of the present invention, the sensors can be used to determine sleep onset. A sleep detection algorithm would preferably include signals from one or all of the following: an accelerometer, a heart rate sensor and a core body temperature sensor. With reference to Fig. 11, a sleep detection indicator signal has three possible states: 1) asleep; 2) awake resting; and 3) awake.

Sleep onset could be determined by, for example, looking for a combination of three factors: low accelerometer based activity, lowered heart rate, and a lowered core body temperature below baseline. The threshold should be calibrated to the individual, based on a resting heart rate determined in a clinic, for example. Detection of a resting but not sleeping state is beneficial. The baseline should not be gathered right before bedtime because circadian rhythms (melatonin levels) cause the CBT and heart rate to go down prior to sleep. The device can be auto calibrated, where the device is set to have a "calibration week" following implant where the baseline resting body temperature and heart rate are determined by the device by measuring these parameters during detected periods over the calibration week. The device may also measure sleeping heart rate and body temperature, or assume a delta based on the general population. The device auto calibration may consist of the following, for example:

1) If the accelerometer output is below a threshold, and the time is not during the patient's normal sleeping hours, the device will measure resting HR and resting CBT, and these values will be averaged over 7 days.
2) If the accelerometer output again is below a threshold, and the time is during the patient's normal sleeping hours, the device will measure sleeping HR and sleeping CBT, and these values will be averaged over 7 days.

Thus, it may be useful to detect a resting state prior to detection of sleep onset for better accuracy, since that is a natural physiological progression. In fact, each time span (i.e., a time span in a range from about 1 minute to about 1 hour) in a 24 hour period may be categorized for a subject according to Awake-Non-Resting, Awake-Resting, and Asleep, as shown in Fig. 11.

A measure of sleep disturbance may be determined for the purpose of early detection of sleep apnea, and for measure of the general quality of the sleep (i.e. average hours of sleep per night, and occurrence of insomnia, or restlessness). This is important for obese patients because sleep deprivation reduces the body's ability to detect fullness.

A Sleep Disturbance Index may be calculated for each time span (i.e. 15 minutes) during sleep based on heart rate and activity parameters: Sleep Disturbance Index = A * (HR - HR_sleep) + B * (Accelerometer based Activity), where A and B are constants determined from a study using clinical data. Alternatively, a Sleep Disturbance Index may be determined for each time span based on three levels of sleep disturbance for the purposes of a diagnostic as listed below:

1) No disturbance;
2) Low level sleep disturbance: increase in activity but no increase in HR;
3) High level of sleep disturbance: increase in activity and increase in HR; and
4) Detection of an awake state.

Then the overall sleep diagnostic for the night would be based on the percent of time spans during sleep that each level of sleep disturbance occurred.

When using activity alone, differentiation of awake versus sleep is generally done based on the length of time that the activity was higher than threshold. This may be done based on a window average, where the window average may be weighted to emphasize recent activity.

If sleep detection is being performed in "real time", then the algorithm could use the current 1 min span, and the four 1 minute spans prior, where the spans were weighted based on temporal distance from the current 1 minute span. If the sleep detection is being performed in a post-processing mode, then the time spans following the 1 minute span of interest may also be used and weighted according to their temporal distance from the span of interest. In addition, heart rate (HR) and core body temperature (CBT) could be included in the determination in a similar way. In the case of HR, one would want to normalize it, by using the HR difference in beats per minute (bpm) from the known or estimated average sleeping heart rate.

In addition, clinical data with known sleep/wake states could be used to determine an index that is easily thresholded to determine sleep versus wake state. All the parameters, HR, accelerometer based activity, and CBT would be averaged over a chosen time span (i.e. 1 - 15 minutes). For example: Sleep Index = C * (HR - RHR) + D * (Accelerometer based Activity) + E * (CBT - CBT_Sleeping). Where, If Sleep Index > Sleep_threshold, subject is awake. If Sleep Index < Sleep_threshold, subject is asleep. Sleep_threshold may be determined from individual calibration or statistical analysis of clinical data.

**Other Therapeutic Sensor-Based Determinations:**

Wellness Diagnostic Based on a Triaxial Accelerometer and Heart Rate (HR) and Core Body Temperature (CBT):

Another embodiment of the present invention includes a general wellness diagnostics based on accelerometer, HR and CBT and factors such as duration and quality of sleep, emotional state/stress level, presence of fever, and metabolism.

Regular and sufficient sleep is very important for good physical and emotional wellbeing. In addition to being a cause for weight gain, insufficient sleep can lead to depression, increased stress, and hypertension. It is possible to determine the duration and quality of sleep and present that as a display or treatment plan diagnostic to a patient and/or health care professional.

Emotional state and stress level have been shown to be correlated with heart rate variability (HRV), respiration rate and blood pressure. HRV, respiration rate, and/or blood pressure could be used either alone or in combination as a diagnostic for the general health, as well as stress level of the patient. In addition, the detection of normal levels of activity is a good indicator of emotional wellbeing. A combination of HRV and daily activity level could be used to provide an indicator of emotional state, stress level, and/or metabolic level. This diagnostic could be in the form of a 1-5 score, where the emotional states are defined along a gradient between negative outlook and high stress to positive outlook and low stress.

The presence of a fever may be detected using a measurement of core body temperature. The presence of illness or frequency of illness may be incorporated into a wellness diagnostic.

Metabolism varies with wake/sleep state, food ingestion, and activity. As discussed above, CBT may be used as an indicator of a subject's metabolism level. Resting metabolism increases with health and fitness. The 24 hour monitoring of CBT will allow an assessment of resting metabolism that can be used to provide a diagnostic related to physical fitness and over all health.

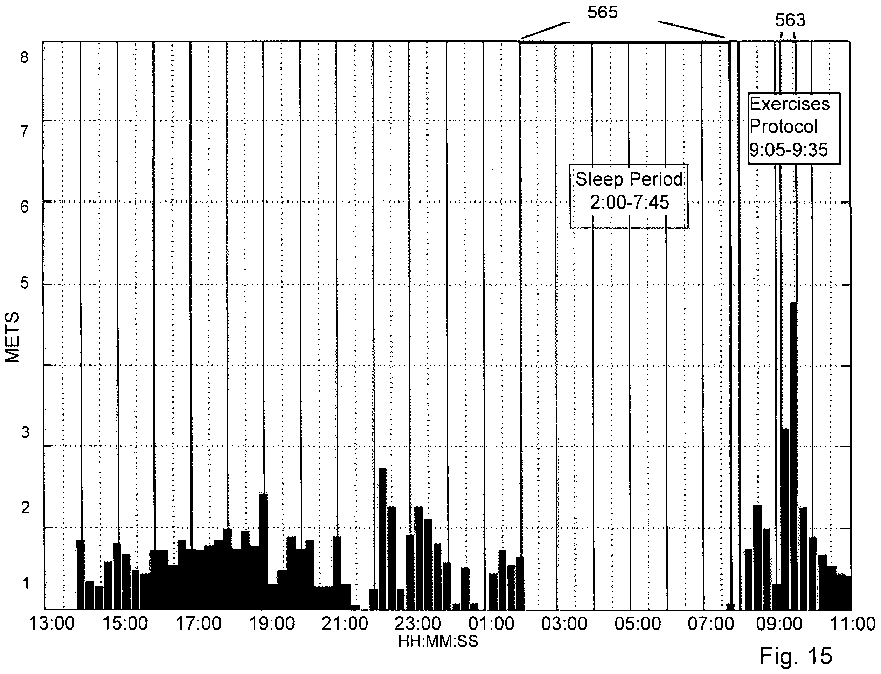

Referring now to Fig. 15, example of an activity trend line over a 24 hour period for a patient with a device and/or system as described herein is shown. In this example, activity intensity is averaged over 15 minute periods, and the activity for each period is represented by an associated bar, with the bar heights varying with activity. A period of relatively high exercise level activity 563 is indicated and identified. During this period the activity intensity is at 3 METS or greater. There is also a period of sleep 565 which can be distinguished by more than 3 sequential 15 minute period at 1.0 METS.

While exemplary embodiments have been described in some detail for clarity of understanding and by way of example, a variety of adaptations, modifications, and changes will be obvious to those of skill in the art. Hence, the scope of the present invention is limited solely by the appended claims.

**Claims**

1. A system for treating an eating disorder of a patient, the system comprising:

   sensors (24a, 24b, 25, 26) implantable in the body of the patient, the implantable sensors comprising an accelerometer coupleable to the body so as to generate accelerometer signals in response to movement of the body, a heart rate sensor coupleable to the body so as to generate heart rate signals in response to a heart rate of the body, a core body temperature sensor coupleable to the body so as to generate temperature signals in response to a core temperature of the body, and a heat flux sensor configured to be located below the epidermis to measure power dissipating through the skin;
   wherein the system is configured to sample the accelerometer signals using a first rate and a second rate and to increase sampling from the first rate to the second rate when the accelerometer output exceeds a predetermined threshold; and

a processor coupleable to the implantable sensors, the processor being configured: to calculate an overall caloric energy expenditure of the body for a time span using the signals by, for each of a plurality of time portions of the span, identifying an associated energy expenditure rate from the signals; and to display the calculated overall caloric energy expenditure on a display.

2. The system of claim 1, wherein the sensor data includes ingestion and activity level information.

3. The system of claim 2, wherein the processor is configured to calculate overall energy expenditure of the body by identifying a plurality of activity levels of the body from the signals, wherein the identified activity levels include sleep and wherein a sleep period is determined using the temperature signals.

4. The system of claim 3, wherein the processor is configured to identify a plurality of activity levels of the body from the signals by determining sleep onset based on reduced levels of accelerometer based activity, heart rate, and core body temperature.

5. The system of claim 1, wherein the accelerometer comprises an omni-directional accelerometer so as to provide greater sensitivity in detecting periods of low activity.

6. The system of claim 1, further comprising stimulation circuitry (116) for providing therapeutic stimulation.

7. The system of claim 1, including a treatment signal applicator attached to the processor to receive overall energy expenditure data from the processor and a stimulator (21) to stimulate tissues of the body to inhibit unhealthy ingestion in response to the signals.

8. The system of claim 7, wherein the stimulator (21) is arranged to be positioned in electrical contact with the stomach and to connect to the processor.

9. The system of claim 7, wherein the stimulator comprises an implantable pulse generator and electrodes (22 and 23) implantable into the stomach muscle layer.

10. The system of any preceding claim, wherein a said sensor is attached to an implantable pulse generator and arranged to be located separately on the stomach wall.

11. The system of any preceding claim, wherein the temperature sensor is arranged to be disposed in the stomach cavity to serve as an ingestion sensor.

**Patentansprüche**

1. System zum Behandeln einer Essstörung bei einem Patienten, wobei das System Folgendes umfasst:

Sensoren (24a, 24b, 25, 26), die im Körper des Patienten implantiert werden können, wobei die implantierbaren Sensoren einen Beschleunigungsmesser umfassen, der so mit dem Körper gekoppelt werden kann, dass er Beschleunigungsmessersignale als Reaktion auf Bewegungen des Körpers erzeugt, einen Herzfrequenzsensor, der so mit dem Körper gekoppelt werden kann, dass er Herzfrequenzsignale als Reaktion auf eine Herzfrequenz des Körpers erzeugt, einen Körperkerntemperatursensor, der so mit dem Körper gekoppelt werden kann, dass er Temperatursignale als Reaktion auf eine Kerntemperatur des Körpers erzeugt, und einen Wärmestromsensor, der zum Positionieren unterhalb der Epidermis konfiguriert ist, um durch die Haut abgeführte Energie zu messen; wobei das System zum Abtasten der Beschleunigungsmessersignale mit einer ersten Rate und einer zweiten Rate und zum Erhöhen der Abtastung von der ersten Rate auf die zweite Rate konfiguriert ist, wenn der Beschleunigungsmesserausgang eine vorbestimmte Schwelle übersteigt; und

einen Prozessor, der mit den implantierbaren Sensoren gekoppelt werden kann, wobei der Prozessor konfiguriert ist zum Berechnen eines kalorischen Gesamtenergieverbrauchs des Körpers für eine Zeitspanne anhand der Signale, indem er für jeden von mehreren Zeitabschnitten der Spanne eine assoziierte Energieverbrauchsrate von den Signalen identifiziert; und den berechneten kalorischen Gesamtenergieverbrauch auf einem Display anzeigt.

**2.** System nach Anspruch 1, wobei die Sensordaten Verdauungs- und Aktivitätsniveauinformationen beinhalten.

**3.** System nach Anspruch 2, wobei der Prozessor zum Berechnen des Gesamtenergieverbrauchs des Körpers konfiguriert ist, indem er mehrere Aktivitätsniveaus des Körpers anhand der Signale identifiziert, wobei die identifizierten Aktivitätsniveaus Schlaf beinhalten und wobei eine Schlafperiode anhand der Temperatursignale bestimmt wird.

**4.** System nach Anspruch 3, wobei der Prozessor zum Identifizieren mehrerer Aktivitätsniveaus des Körpers anhand der Signale konfiguriert ist, durch Ermitteln des Einschlafens auf der Basis von reduzierten Niveaus von beschleunigungsmesserbasierter Aktivität, Herzfrequenz und Körperkerntemperatur.

**5.** System nach Anspruch 1, wobei der Beschleunigungsmesser einen omnidirektionalen Beschleunigungsmesser umfasst, um eine größere Empfindlichkeit beim Erkennen von Perioden von niedriger Aktivität bereitzustellen.

**6.** System nach Anspruch 1, das ferner Stimulationsschaltungen (116) zum Bereitstellen von therapeutischer Stimulation umfasst.

**7.** System nach Anspruch 1, das einen Behandlungssignalapplikator beinhaltet, der an dem Prozessor angebracht ist, um Gesamtenergieverbrauchsdaten von dem Prozessor zu erhalten, und einen Stimulator (21) zum Stimulieren von Geweben des Körpers, um ungesunde Aufnahme zu hemmen, als Reaktion auf die Signale.

**8.** System nach Anspruch 7, wobei der Stimulator (21) zum Positionieren in elektrischem Kontakt mit dem Bauch und zum Verbinden mit dem Prozessor ausgelegt ist.

**9.** System nach Anspruch 7, wobei der Stimulator einen implantierbaren Pulsgenerator und Elektroden (22 und 23) umfasst, die in der Bauchmuskelschicht implantiert werden können.

**10.** System nach einem vorherigen Anspruch, wobei ein genannter Sensor an einem implantierbaren Pulsgenerator angebracht und zum separaten Positionieren an der Bauchwand ausgelegt ist.

**11.** System nach einem vorherigen Anspruch, wobei der Temperatursensor zum Anordnen in der Bauchhöhle ausgelegt ist, so dass er als Aufnahmesensor dient.

**Revendications**

**1.** Système de traitement d'un trouble alimentaire d'un patient, le système comprenant :

des capteurs (24a, 24b, 25, 26) implantables dans le corps d'un patient, les capteurs implantables comprenant un accéléromètre pouvant être couplé avec le corps de manière à générer des signaux d'accéléromètre en réponse au mouvement du corps, un capteur de fréquence cardiaque pouvant être couplé au corps de manière à générer des signaux de fréquence cardiaque en réponse à une fréquence cardiaque du corps, un capteur de température corporelle centrale pouvant être couplé au corps de manière à générer des signaux de température en réponse à une température corporelle centrale, et un capteur de flux thermique configuré pour être situé sous l'épiderme pour mesurer la dissipation de puissance à travers la peau ;
dans lequel le système est configuré pour échantillonner les signaux de l'accéléromètres à l'aide d'une première vitesse et d'une deuxième vitesse et pour augmenter l'échantillonnage de la première vitesse à la deuxième vitesse quand la sortie de l'accéléromètre dépasse un seuil prédéterminé ; et
un processeur pouvant être couplé avec les capteurs implantables, le processeur étant configuré : pour calculer une dépense énergétique calorique globale du corps pendant une période de temps à l'aide des signaux, pour chaque portion de temps d'une pluralité de portions de temps de la période, en identifiant un taux de dépense énergétique associée d'après les signaux ; et pour afficher la dépense énergétique calorique globale calculé sur un écran.

**2.** Système de la revendication 1, dans lequel les données du capteur comprennent des informations sur les niveaux d'ingestion et d'activité.

**3.** Système de la revendication 2, dans lequel le processeur est configuré pour calculer la dépense énergétique globale du corps en identifiant une pluralité de niveaux d'activité du corps d'après les signaux, dans lequel les niveaux d'activité identifiés comprennent le sommeil et dans lequel une période de sommeil est déterminée à l'aide des signaux de température.

4.  Système de la revendication 3, dans lequel le processeur est configuré pour identifier une pluralité de niveaux d'activité du corps d'après les signaux en déterminant l'endormissement sur la base de niveaux réduits de l'activité basée sur l'accéléromètre, la fréquence cardiaque, et la température corporelle centrale.

5.  Système de la revendication 1, dans lequel l'accéléromètre comprend un accéléromètre omnidirectionnel de manière à fournir une plus grande sensibilité lors de la détection de périodes de faible activité.

6.  Système de la revendication 1, comprenant en outre une circuiterie de stimulation (116) pour fournir une stimulation thérapeutique.

7.  Système de la revendication 1, comprenant un applicateur de signaux de traitement attaché au processeur pour recevoir des données de dépense énergétique globale du processeur et un stimulateur (21) pour stimuler des tissus du corps pour inhiber une ingestion malsaine en réponse aux signaux.

8.  Système de la revendication 7, dans lequel le stimulateur (21) est agencé pour être positionné en contact électrique avec l'estomac et pour se connecter au processeur.

9.  Système de la revendication 7, dans lequel le stimulateur comprend un générateur de pulsations implantable et des électrodes (22 et 23) implantables dans la couche musculaire de l'estomac.

10.  Système de l'une quelconque des revendications précédentes, dans lequel un dit capteur est attaché à un générateur de pulsations implantable et agencé pour être situé séparément sur la paroi de l'estomac.

11.  Système de l'une quelconque des revendications précédentes, dans lequel le capteur de température est agencé pour être disposé dans la cavité de l'estomac pour servir de capteur d'ingestion.

Fig. 1

Fig. 2

Implant device including sensor in body of patient ~300

In response to eating and/or exercise by the patient, collect patient data with the sensor ~310

Analyze patient data to determine sensor-based information about the patient ~320

Provide the eating and exercise information to promote healthy behavior by the patient ~330

**FIG. 3A**

Provide the eating and exercise information to promote healthy behavior by the patient ~330

Provide remote access to the information ~332

Provide access to the information via the internet ~334

**FIG. 3B**

Provide the eating and exercise information to promote healthy behavior by the patient ~330

Provide a graphical display of the information ~336

**FIG. 3C**

Provide the eating and exercise information to promote healthy behavior by the patient ~330

Display the information via a website ~338

Accept data input to the website by the patient ~340

Analyze input data in conjunction with sensor data ~350

Provide analysis to a user ~360

**FIG. 3D**

```
┌─────────────────────┐
│  Collect data with at least │
│  one sensor at intervals    │ ⟍400
│  over a period of time      │
└─────────────────────┘
          │
          ▼
┌─────────────────────┐
│  Obtain sensor data from    │
│  the implanted sensor(s)    │ ⟍410
└─────────────────────┘
          │
          ▼
┌─────────────────────┐
│  Present the sensor data    │
│  to a user via a graphical  │
│  interface                  │ ⟍420
└─────────────────────┘
```

FIG. 4A

```
┌─────────────────────┐
│  Collect data with at least │
│  one sensor at intervals    │ ⟍400
│  over a period of time      │
└─────────────────────┘
          │
          ▼
┌─────────────────────┐        ┌─────────────────────────┐
│  Obtain sensor data from    │        │  Accept patient-input data      │
│  the implanted sensor(s)    │        │                                 │ ⟍430
└─────────────────────┘        └─────────────────────────┘
   410⟍                                    │
          └──────────────┬─────────────────┘
                         │
                         ▼
          ┌─────────────────────────┐
          │  Present the sensor data        │
          │  and patient-input data to a    │
          │  user via a graphical           │ ⟍440
          │  interface                      │
          └─────────────────────────┘
                         │
                         ▼
          ┌─────────────────────────┐
          │  Compare the sensor data        │
          │  to the patient-input data      │ ⟍450
          └─────────────────────────┘
                         │
                         ▼
          ┌─────────────────────────┐
          │  Provide the comparison         │
          │  information to the user        │ ⟍460
          └─────────────────────────┘
```

FIG. 4B

FIG. 4C

Fig. 5

EP 2 475 296 B1

Fig. 6A

Fig. 6B

Fig. 6C

FIG. 7

YES
(Active)

3.METS=0.6*METS(Hr_based)+
(1-0.6)*METS(Acc_based)

YES
(Indicative of
Stress or
Caffeine
Induced HR↑)

4.METS=0.2*METS(HR_based)
+ (1-0.2)*METS(Acc_based)
[Predominatly activity based,
~20% HR]

1. METS(Acc_based)>2.0

NO
(Sedentary)

2.HR>10 bpm + RHR

NO

5.METS=0.1*METS(HR_based)
+ (1-0.1)*METS(Acc_based)
[Predominatly activity based,
10% HR]

**Definitions:**
METS(HR_based): METS estimation from HR-METS regression equation determined with individual or group calibration.
METS(Acc_based): Where METS is based on IAAtot linear regression or 2 regression eqation model developed by Crouter

FIG. 8

900    901    902A

Lead    Stimulus
Electrode

Return
Electrode
With
Thermistor

LGSxxx

FIG. 9A

903

Thermistor in
Thermally Conductive
Block

LGSxxx

FIG. 9B

Thermistor

902B

902A

LGSxxx

Fig. 9C

904

Thermopile in IR/ Optical Window

LGSxxx

Fig. 9D

```
                                                    ┌──────────────────────────────────┐
                                                    │  4. METS=a*(ΔCBT*HR) +           │
                                          YES (Exercise)│     b*METS(acc_based)        │
                                          ──────────────▶│ (Where regression relationships │
                                                    │   are based on exercise periods  │
                                                    └──────────────────────────────────┘
                              ┌────────────────────────┐
                              │ 2. METS(ΔCBT*HR)>Thresh │
            YES (Active)      │  (Use core body temp to │
      ─────────────────────▶  │ differentiate between   │
                              │ exercise and daily      │
                              │ living activity-NEAT)    │
                              └────────────────────────┘     ┌──────────────────────────────────┐
                                          │                  │  5. METS=a*(ΔCBT*HR) +           │
                                          │ NO (Daily Living │     b*METS(acc_based)            │
                                          │    Activity)     │ (Where regression relationships  │
                                          ──────────────────▶│ are based on daily living activities)│
 ┌─────────────────────┐                                     └──────────────────────────────────┘
 │ 1.METS(Acc_based)>2.0│
 └─────────────────────┘
```

4. METS=a*(ΔCBT*HR) + b*METS(acc_based) (Where regression relationships are based on exercise periods

2. METS(ΔCBT*HR)>Thresh (Use core body temp to differentiate between exercise and daily living activity-NEAT)

YES (Exercise)

YES (Active)

1.METS(Acc_based)>2.0

5. METS=a*(ΔCBT*HR) + b*METS(acc_based) (Where regression relationships are based on daily living activities)

NO (Daily Living Activity)

NO (Subject Appears to Be Sedentary)

3. HR>10 bpm + RHR

NO

7. METS=a*(ΔCBT*HR) + b*METS(acc_based) (Where regression relationships are based on static exercise or post-exercise ↑EE)

Definitions:
METS*(HR_based)*: METS estimation from HR-METS regression equation determined with individual or grou[ calibration.
METS*(Acc_based)*: Where METS is based on IAA$_{tot}$-METS linear regressionor 2 regression equation model developed by Crouter
METS*(ΔCBT*HR)*=METS is based on regression analysis assuming the product of core body temperature and heart rate is proportional to heat energy created

6. METS(ΔCBT*HR)<Thresh (Use core body temp to differentiate between increase in HR due to stress vs. activity or post-exercise ↑EE)

YES (Indicative of Stress or Caffeine HR↑)

NO (Subject Sedentary)

8. METS=0.1*METS(Hr_based) + (1-0.1)*METS(Acc_based) (based predominatly on 3D accelerometer)

FIG. 10

Awake Non-
Resting

Detect Non-Resting

Detect
Resting

Awake
Resting

Detect Awake

Detect Sleep
Onset

Asleep

FIG. 11

FIG. 12A

Heat Flux
Sensor
Below
Epidermis

Vascular
Conductivity (σ)

Blood Specific Heat (Cp)
Blood Mass-Density (ρ)

Convection

Skin

Evaporation

Tcore

Internal
Tissue
(Core)

$P = \Delta E / \Delta t$

Heat
Energy
Flow

Conduction

$P = \Delta E / \Delta t$

T environment

Blood Flow Rate=Heart-Rate *Heart Volume *σ

Heart

Heart
Volume
(Vheart)

Heart-Rate (HR)

Temperature
Sensor
"Coolest"
Blood—
Juncture of
Capillaries
into a Larger
Vein

FIG. 12B

Fig. 13A

EP 2 475 296 B1

$$Q \cdot = \frac{-\Delta T}{Rth^* \Delta X}$$

FIG. 13B

FIG. 14

Fig. 15

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4178105 A
- US 7020531 B
- US 7120498 B
- US 7509174 B
- US 7509175 B
- US 7054690 B
- US 7430450 B

- US 20070173705 A
- US 20070255334 A
- US 61166636 A
- US 14543008 A
- US 95034504 A
- US 61122315 A

**Non-patent literature cited in the description**

- **COMMITTEE, P.A.G.A.** Physical Activity Guidelines Advisory Committee Report, 2008. U.S.D.o.H.a.H. Services, 2008
- **BOUTEN, C.** A Triaxial Accelerometer and Portable Data Processing Unit for the Assessment of Daily Physical Activity. *IEEE Transactions on Biomedical Engineering,* 1997, vol. 44 (3
- **CHEN, K.Y. ; M. SUN.** Improving energy expenditure estimation by using a triaxial accelerometer. *J Appl Physiol,* 1997, vol. 83 (6), 2112-22
- **PLASQUI, G. et al.** Measuring free-living energy expenditure and physical activity with triaxial accelerometry. *Obes Res,* 2005, vol. 13 (8), 1363-9
- **SCHUTZ, Y. et al.** A new accelerometric method to assess the daily walking practice. *Int J Obes Relat Metab Disord,* 2002, vol. 26 (1), 111-8
- **MCCRORY, M.A. et al.** Between-day and within-day variability in the relation between heart rate and oxygen consumption: effect on the estimation of energy expenditure by heart-rate monitoring. *Am J Clin Nutr,* 1997, vol. 66 (1), 18-25
- **STRATH, S.J. et al.** Evaluation of heart rate as a method for assessing moderate intensity physical activity. *Med Sci Sports Exerc,* 2000, vol. 32 (9), 465-70
- **CEESAY, S.M. et al.** The use of heart rate monitoring in the estimation of energy expenditure: a validation study using indirect whole-body calorimetry. *Br J Nutr,* 1989, vol. 61 (2), 175-86
- **CROUTER, S.E. ; J.R. CHURILLA ; D.R. BASSETT, JR.** Accuracy of the for the assessment of energy expenditure in adults. *Eur J Clin Nutr,* 2008, vol. 62 (6), 704-11
- **MOON, J.K. ; N.F. BUTTE.** Combined heart rate and activity improve estimates of oxygen consumption and carbon dioxide production rates. *J Appl Physiol,* 1996, vol. 81 (4), 1754-61

- **STRATH, S.J. et al.** Simultaneous heart rate-motion sensor technique to estimate energy expenditure. *Med Sci Sports Exerc,* 2001, vol. 33 (12), 2118-23
- **STRATH, S.J. et al.** Validity of the simultaneous heart rate-motion sensor technique for measuring energy expenditure. *Med Sci Sports Exerc,* 2002, vol. 34 (5), 888-94
- **TREUTH, M.S. ; A.L. ADOLPH ; N.F. BUTTE.** Energy expenditure in children predicted from heart rate and activity calibrated against respiration calorimetry. *Am J Physiol,* 1998, vol. 275 (1), E 12-8
- **J AKICIC, J.M.** Evaluation of the Sense Wear Pro Armband to assess energy expenditure during exercise. *Med Sci Sports Exerc,* 2004, vol. 36 (5), 897-904
- **ST-ONGE, M. et al.** Evaluation of a portable device to measure daily energy expenditure in free-living adults. *Am J Clin Nutr,* 2007, vol. 85 (3), 742-9
- Evaluation of a multisensor armband in estimating energy expenditure in obese individuals. **PAPAZOGLOU, D. et al.** Obesity. Silver Spring, 2006, vol. 14, 2217-23
- **BOUTEN, C.V. et al.** Assessment of energy expenditure for physical activity using a triaxial accelerometer. *Med Sci Sports Exerc,* 1994, vol. 26 (12), 1516-23
- **PATE, R.R. et al.** Physical activity and public health. A recommendation from the Centers for Disease Control and Prevention and the American College of Sports Medicine. *JAMA,* 1995, vol. 273 (5), 402-7
- **LEVINE, J.A. et al.** Jnterindividual variation in posture allocation: possible role in human obesity. *Science,* 2005, vol. 307 (5709), 584-6
- **MAC WILLIAM.** Postural Effects on Heart-Rate and Blood-Pressure. *Exp Physiol,* 1933, XXIII

- **CROUTER, S.E. ; D.R. BASSETT, JR.** A new 2-regression model for the Actical accelerometer. *Br J Sports Med,* 2008, vol. 42 (3), 217-24
- **KIM, D. et al.** Detection of subjects with higher self-reporting stress scores using heart rate variability patterns during the day. *Conf Proc IEEE Eng Med Biol Soc,* 2008, 682-5
- **YOSHIUCHI, K. et al.** Yearlong physical activity and depressive symptoms in older Japanese adults: cross-sectional data from the Nakanojo study. *Am J Geriatr Psychiatry,* 2006, vol. 14 (7), 621-4
- **BRAGE, S. et al.** Branched equation modeling of simultaneous accelerometery and heart rate monitoring improves estimate of directly measured physical activity energy expenditure. *J Appl Physiol,* 2004, vol. 96, 343-351